# EUROPEAN PATENT APPLICATION

(11) **EP 2 141 227 A2**
(43) Date of publication of application: **06.01.2010**
(21) Application number: 09165364.2
(22) Date of filing: 08.02.2002
(51) Int. Cl.: C12N 1/21, C07K 14/22, C07K 14/285, C07K 14/195, A61K 39/095, A61K 39/10, A61K 39/102, A61K 39/104, A61K 39/106

(54) **Hyperblebbing bacterial strains and use thereof for production of vaccines**

(30) Priority: 08.02.2001 GB 0103171
(62) Divisional of application: 02700219.5
(71) Applicant: SmithKline Beecham Biologicals S.A., 1330 Rixensart (DE)
(72) Inventor: Berthet, Francois-Xavier Jacques, 1330 Rixensart (BE); Denoel, Philippe, 1330 Rixensart (BE); Neyt, Cecile Anne, 1330 Rixensart (BE); Poolman, Jan, 1330 Rixensart (BE); Thonnard, Joelle, 1330 Rixensart (BE)
(74) Representative: Lubienski, Michael John

(57) **Abstract**

The present invention relates to the field of, engineered Gram-negative bacterial strains that have outer-membrane vesicle shedding properties, and vaccine compositions comprising these bacteria or vesicles. The present invention provides a hyperblebbing Gram-negative bacterium which has been genetically modified by either or both processes selected from a group consisting of: down- regulation of expression of one or more *tol* genes; and mutation of one or more gene(s) encoding a protein comprising a peptidoglycan-associated site to attenuate the peptidoglycan-binding activity of the protein(s).

## Description

### FIELD OF THE INVENTION

The present invention relates to the field of Gram-negative bacterial vaccine compositions, their manufacture, and the use of such compositions in medicine. More particularly it relates to the field of novel, engineered Gram-negative bacterial strains that have improved outer-membrane vesicle shedding properties, and vaccine compositions comprising these vesicles.

### BACKGROUND OF THE INVENTION

Gram-negative bacteria are separated from the external medium by two successive layers of membrane structures. These structures, referred to as the cytoplasmic membrane and the outer membrane (OM), differ both structurally and functionally. The outer membrane plays an important role in the interaction of pathogenic bacteria with their respective hosts. Consequently, the surface exposed bacterial molecules represent important targets for the host immune response, making outer-membrane components attractive candidates in providing vaccine, diagnostic and therapeutics reagents.

Whole cell bacterial vaccines (killed or attenuated) have the advantage of supplying multiple antigens in their natural micro-environment. Drawbacks around this approach are the side effects induced by bacterial components such as endotoxin and peptidoglycan fragments. On the other hand, acellular subunit vaccines containing purified components from the outer membrane may supply only limited protection and may not present the antigens properly to the immune system of the host.

Proteins, phospholipids and lipopolysaccharides are the three major constituents found in the outer-membrane of all Gram-negative bacteria. These molecules are distributed asymmetrically: membrane phospholipids (mostly in the inner leaflet), lipooligosaccharides (exclusively in the outer leaflet) and proteins (inner and outer leaflet lipoproteins, integral or polytopic membrane proteins). For many bacterial pathogens which impact on human health, lipopolysaccharide and outer-membrane proteins have been shown to be immunogenic and amenable to confer protection against the corresponding disease by way of immunization.

The OM of Gram-negative bacteria is dynamic and, depending on the environmental conditions, can undergo drastic morphological transformations. Among these manifestations, the formation of outer-membrane vesicles or "blebs" has been studied and documented in many Gram-negative bacteria (Zhou, Let al. 1998. FEMS Microbiol. Lett.163: 223-228). Among these, a non-exhaustive list of bacterial pathogens reported to produce blebs include: *Bordetella pertussis, Borrelia burgdorferi, Brucella melitensis, Brucella ovis, Chlamydia psittaci, Chlamydia trachomatis, Esherichia coli, Haemophilus influenzae, Legionella pneumophila, Neisseria gonorrhoeae, Neisseria meningitidis, Pseudomonas aeruginosa* and *Yersinia enterocolitica.* Although the biochemical mechanism responsible for the production of OM blebs is not fully understood, these outer membrane vesicles have been extensively studied as they represent a powerful methodology in order to isolate outer-membrane protein preparations in their native conformation. In that context, the use of outer-membrane preparations is of particular interest to develop vaccines against *Neisseria, Moraxella catarrhalis, Haemophilus influenzae, Pseudomonas aeruginosa* and *Chlamydia.* Moreover, outer membrane blebs combine multiple proteinaceaous and non-proteinaceous antigens that are likely to confer extended protection against intra-species variants.

Examples of bacterial species from which bleb vaccines can be made are the following.

### Neisseria meningitidis:

*Neisseria meningitidis* (meningococcus) is a Gram-negative bacterium frequently isolated from the human upper respiratory tract. It occasionally causes invasive bacterial diseases such as bacteremia and meningitis. The incidence of meningococcal disease shows geographical seasonal and annual differences (Schwartz, B., Moore, P.S., Broome, C.V.; Clin. Microbiol. Rev. 2 (Supplement), S18-S24, 1989). Most disease in temperate countries is due to strains of serogroup B and varies in incidence from 1-10/100,000/year total population sometimes reaching higher values (Kaczmarski, E.B. (1997), Commun. Dis. Rep. Rev. 7: R55-9, 1995; Scholten, R.J.P.M., Bijlmer, H.A., Poolman, J.T. et al. Clin. Infect. Dis. 16: 237-246, 1993; Cruz, C., Pavez, G., Aguilar, E., et al. Epidemiol. Infect. 105: 119-126, 1990). Age-specific incidences in the two high risk-groups, infants and teenagers, reach higher levels.

Epidemics dominated by serogroup A meningococci occur, mostly in central Africa, sometimes reaching levels up to 1000/100,000/year (Schwartz, B., Moore, P.S., Broome, C.V. Clin. Microbiol. Rev. 2 (Supplement), S18-S24, 1989). Nearly all cases of meningococcal disease as a whole are caused by serogroup A, B, C, W-135 and Y meningococci. A tetravalent A, C, W-135, Y capsular polysaccharide vaccine is available (Armand, J., Arminjon, F., Mynard, M.C., Lafaix, C., J. Biol. Stand. 10; 335-339, 1982).

The polysaccharide vaccines are currently being improved by way of chemically conjugating them to carrier proteins (Lieberman, J.M., Chiu, S.S., Wong, V.K., et al. JAMA 275 : 1499-1503, 1996). A serogroup B vaccine is not available, since the B capsular polysaccharide is non-immunogenic, most likely because it shares structural similarity to host components (Wyle, F.A., Artenstein, M.S., Brandt, M.L. et al. J. Infect. Dis. 126: 514-522, 1972; Finne, J.M., Leinonen, M., Mäkelä, P.M. Lancet ii.: 355-357, 1983).

For many years efforts have been focused on developing meningococcal outer membrane based vaccines (de Moraes, J.C., Perkins, B., Camargo, M.C. et al. Lancet 340: 1074-1078, 1992; Bjune, G., Hoiby, E.A. Gronnesby, J.K. et al. 338: 1093-1096, 1991). Such vaccines have demonstrated efficacies from 57% - 85% in older children (>4 years) and adolescents. Most of these efficacy trials were performed with OMV (outer membrane vesicles, derived by LPS depletion from blebs) vaccines derived from wild-type *N. meningitidis* B strains.

*N. meningitidis* serogroup B (menB) excretes outer membrane blebs in quantities that allow their preparation on an industrial scale. Such multicomponent outer-membrane protein vaccines from naturally-occurring menB strains have been found to be efficacious in protecting teenagers from menB disease and have become registered in Latin America. An alternative method of preparing outer-membrane vesicles is via the process of detergent extraction of the bacterial cells (EP 11243).

Many bacterial outer membrane components are present in these vaccines, such as PorA, PorB, Rmp, Opc, Opa, FrpB and the contribution of these components to the observed protection still needs further definition. Other bacterial outer membrane components have been defined (using animal or human antibodies) as potentially being relevant to the induction of protective immunity, such as TbpB, NspA (Martin, D., Cadieux, N., Hamel, J., Brodeux, B.R., J. Exp. Med. 185: 1173-1183, 1997; Lissolo, L., Maitre-Wilmotte, C., Dumas, p. et al., Inf. Immun. 63: 884-890, 1995). The mechanism of protective immunity will involve antibody mediated bactericidal activity and opsonophagocytosis.

### Moraxella catarrhalis

*Moraxella catarrhalis* (also named *Branhamella catarrhalis)* is a Gram-negative bacterium frequently isolated from the human upper respiratory tract. It is responsible for several pathologies, the main ones being otitis media in infants and children, and pneumonia in the elderly. It is also responsible for sinusitis, nosocomial infections and, less frequently, for invasive diseases.

Bactericidal antibodies have been identified in most adults tested (Chapman, AJ et al. (1985) J. Infect.Dis. 151:878). Strains of *M. catarrhalis* present variations in their capacity to resist serum bactericidal activity: in general, isolates from diseased individuals are more resistant than those who are simply colonized (Hol, C et al. (1993) Lancet 341:1281, Jordan, KL et al. (1990) Am.J.Med. 88 (suppl. 5A):28S). Serum resistance could therfore be considered as a virulence factor of the bacteria. An opsonizing activity has been observed in the sera of children recovering from otitis media.

The antigens targetted by these different immune responses in humans have not been identified, with the exception of OMP B1, a 84 kDa protein, the expression of which is regulated by iron, and that is recognized by the sera of patients with pneumonia (Sethi, S, et al. (1995) Infect.Immun. 63:1516), and of UspA1 and UspA2 (Chen D. et al.(1999), Infect.Immun. 67:1310).

A few other membrane proteins present on the surface of *M. catarrhalis* have been characterized using biochemical methods for their potential implication in the induction of a protective immunity (for review, see Murphy, TF (1996) Microbiol.Rev. 60:267). In a mouse pneumonia model, the presence of antibodies raised against some of them (UspA, CopB) favors a faster clearance of the pulmonary infection. Another polypeptide (OMP CD) is highly conserved among *M. catarrhalis* strains, and presents homologies with a porin *of Pseudomonas aeruginosa,* which has been demonstrated to be efficacious against this bacterium in animal models.

*M. catarrhalis* produces outer membrane vesicles (Blebs). These Blebs have been isolated or extracted by using different methods. Among these methods, detergent extraction (Bartos L.C. and Murphy T.M. 1988. J. Infect. Dis. 158: 761-765; Murphy T.M. and Loeb M.R. 1989 Microbial Pathog. 6:159-174; Unhanand M., Maciver I., Ramilo O., Arencibia-Mireles O.,Argyle J.C., McCracken G.H., Hansen E.J. 1992. J. Infect. Dis. 165: 644-650; Maciver I., Unhanand M., McCracken G.H. and Hansen E.J. 1993. J. Infect. Dis. 168: 469-472) or the production of ghosts (Lubitz W., et al. 1999. J. Biotechnol. 73: 261-273; Eko F.O., et. al. 1999. Vaccine 17: 1643-1649) are well known. The protective capacity of such Bleb preparations has been tested in a murine model for pulmonary clearance of *M. catarrhalis.* It has been shown that active immunization with Bleb vaccine or passive transfer of anti-Blebs antibody induces significant protection in this model (Maciver I., Unhanand M., McCracken G.H. Jr., Hansen, E.J. 1993. J. Infect. Dis. 168: 469-472).

### Haemophilus influenzae

*Haemophilus influenzae* is a non-motile Gram-negative bacterium. Man is its only natural host. *H. influenzae* isolates are usually classified according to their polysaccharide capsule. Six different capsular types designated 'a' through 'f' have been identified. Isolates that fail to agglutinate with antisera raised against one of these six serotypes are classified as nontypeable, and do not express a capsule.

*H. influenzae* type b (Hib) is clearly different from the other types in that it is a major cause of bacterial meningitis and systemic diseases. Nontypeable strains of *H*. *influenzae* (NTHi) are only occasionally isolated from the blood of patients with systemic disease. NTHi is a common cause of pneumonia, exacerbation of chronic bronchitis, sinusitis and otitis media. NTHi strains demonstrate a large variability as identified by clonal analysis, whilst Hib strains as a whole are more homogeneous.

Various proteins of *H. influenzae* have been shown to be involved in pathogenesis or have been shown to confer protection upon vaccination in animal models.

Adherence of NTHi to human nasopharygeal epithelial cells has been reported (Read RC. et al. 1991. J. Infect. Dis. 163:549). Apart from fimbriae and pili (Brinton CC. et al. 1989. Pediatr. Infect. Dis. J. 8:S54; Kar S. et al. 1990. Infect. Immun. 58:903; Gildorf JR. et al. 1992. Infect. Immun. 60:374; St. Geme JW et al. 1991. Infect. Immun. 59:3366; St. Geme JW et al. 1993. Infect. Immun. 61: 2233), many adhesins have been identified in NTHi. Among them, two surface exposed high-molecular-weight proteins designated HMW1 and HMW2 have been shown to mediate adhesion of NTHi to epithelial cells (St. Geme JW. et al. 1993. Proc. Natl. Acad. Sci. USA 90:2875). Another family of high-molecular-weight proteins has been identified in NTHi strains that lack proteins belonging to BAlW1/HMW2 family. The NTHi 115-kDa Hia protein (Barenkamp SJ., St Geme S.W. 1996. Mol. Microbiol. In press) is highly similar to the Hsf adhesin expressed by *H. influenzae* type b strains (St. Geme JW. et al. 1996. J. Bact. 178:6281). Another protein, the Hap protein shows similarity to IgA1 serine proteases and has been shown to be involved in both adhesion and cell entry (St. Geme JW. et al. 1994. Mol. Microbiol. 14:217).

Five major outer membrane proteins (OMP) have been identified and numerically numbered. Original studies using *H.influenzae* type b strains showed that antibodies specific for P1 and P2 OMPs protected infant rats from subsequent challenge (Loeb MR. et al. 1987. Infect. Immun. 55:2612; Musson RS. Jr. et al. 1983. J. Clin. Invest. 72:677). P2 was found to be able to induce bactericidal and opsonic antibodies, which are directed against the variable regions present within surface exposed loop structures of this integral OMP (Haase EM. et al. 1994 Infect. Immun. 62:3712; Troelstra A. et al. 1994 Infect. Immun. 62:779). The lipoprotein P4 also may induce bactericidal antibodies (Green BA. et al. 1991. Infect.Immun.59:3191).

OMP P6 is a conserved peptidoglycan associated lipoprotein making up 1-5 % of the outer membrane (Nelson MB. et al. 1991. Infect. Immun. 59:2658). Later a lipoprotein of about the same molecular weight was recognized called PCP (P6 cross-reactive protein) (Deich RM. et al. 1990. Infect. Immun. 58:3388). A mixture of the conserved lipoproteins P4, P6 and PCP did not reveal protection as measured in a chinchilla otitis-media model (Green BA. et al. 1993. Infect.immun. 61:1950). P6 alone appears to induce protection in the chinchilla model (Demaria TF. et al. 1996. Infect. Immun. 64:5187).

A fimbrin protein (Miyamoto N., Bakaletz, LO. 1996. Microb. Pathog. 21:343) has also been described with homology to OMP P5, which itself has sequence homology to the integral *Escherichia coli* OmpA (Miyamoto N., Bakaletz, LO. 1996. Microb. Pathog. 21:343; Munson RS. Jr. et al. 1993. Infect. Immun. 61:1017). NTHi seem to adhere to mucus by way of fimbriae.

In line with the observations made with gonococci and meningococci, NTHi expresses a dual human transferrin receptor composed of TbpA and TbpB when grown under iron limitation. Anti-TbpB antibody protected infant rats (Loosmore SM. et al. 1996. Mol. Microbiol. 19:575). Hemoglobin / haptoglobin receptor also have been described for NTHi (Maciver I. et al. 1996. Infect. Immun. 64:3703). A receptor for Haem:Hemopexin has also been identified (Cope LD. et al. 1994. Mol.Microbiol. 13:868). A lactoferrin receptor is also present amongst NTHi, but is not yet characterized (Schryvers AB. et al. 1989. J. Med. Microbiol. 29:121). A protein similar to neisserial FrpB-protein has not been described amongst NTHi.

An 80kDa OMP, the D15 surface antigen, provides protection against NTHi in a mouse challenge model. (Flack FS. et al. 1995. Gene 156:97). A 42kDa outer membrane lipoprotein, LPD is conserved amongst *Haemophilus influenzae* and induces bactericidal antibodies (Akkoyunlu M. et al. 1996. Infect. Immun. 64:4586). A minor 98kDa OMP (Kimura A. et al. 1985. Infect. Immun. 47:253), was found to be a protective antigen, this OMP may very well be one of the Fe-limitation inducible OMPs or high molecular weight adhesins that have been characterized thereafter. *H. Influenzae* produces IgA1-protease activity (Mulks MH., Shoberg RJ. 1994. Meth. Enzymol. 235:543). IgA1-proteases of NTHi have a high degree of antigenic variability (Lomholt H., van Alphen L., Kilian, M. 1993. infect. Immun. 61:4575).

Another OMP of NTHi, OMP26, a 26-kDa protein has been shown to enhance pulmonary clearance in a rat model (Kyd, J.M., Cripps, A.W. 1998. Infect. Immun. 66:2272). The NTHi HtrA protein has also been shown to be a protective antigen. Indeed, this protein protected Chinchilla against otitis media and protected infant rats against *H. influenzae* type b bacteremia (Loosmore S.M. et al. 1998. Infect. Immun. 66:899).

Outer membrane vesicles (or blebs) have been isolated from *H. influenzae* (Loeb M.R., Zachary A.L., Smith D.H. 1981. J. Bacteriol. 145:569-604; Stull T.L., Mack K., Haas J.E., Smit J., Smith A.L. 1985. Anal. Biochem. 150: 471-480), as have the production of ghosts (Lubitz W., et al. 1999. J. Biotechnol. 73: 261-273; Eko F.O:, et. al. 1999. Vaccine 17: 1643-1649). The vesicles have been associated with the induction of blood-brain barrier permeability (Wiwpelwey B., Hansen E.J., Scheld W.M. 1989 Infect. Immun. 57: 2559-2560), the induction of meningeal inflammation (Mustafa M.M., Ramilo O., Syrogiannopoulos G.A., Olsen K.D., McCraken G.H. Jr., Hansen, E.J. 1989. J. Infect. Dis. 159: 917-922) and to DNA uptake (Concino M.F., Goodgal S.H. 1982 J. Bacteriol. 152: 441-450). These vesicles are able to bind and be absorbed by the nasal mucosal epithelium (Harada T., Shimuzu T., Nishimoto K., Sakakura Y. 1989. Acta Otorhinolarygol. 246: 218-221) showing that adhesins and/or colonization factors could be present in Blebs. Immune response to proteins present in outer membrane vesicles has been observed in patients with various *H. influenzae* diseases (Sakakura Y., Harada T., Hamaguchi Y., Jin C.S. 1988. Acta Otorhinolarygol. Suppl. (Stockh.) 454: 222-226; Harada T., Sakakura Y., Miyoshi Y. 1986. Rhinology 24: 61-66).

### Pseudomonas aeruginosa:

The genus *Pseudomonas* consists of Gram-negative, polarly flagellated, straight and slightly curved rods that grow aerobically and do not forms spores. Because of their limited metabolic requirements, *Pseudomonas spp.* are ubiquitous and are widely distributed in the soil, the air, sewage water and in plants. Numerous species of *Pseudomonas* such as *P. aeruginosa, P. pseudomallei, P. mallei, P. maitopnilia and P. cepacia* have also been shown to be pathogenic for humans. Among this list, *P. aeruginosa* is considered as an important human pathogen since it is associated with opportunistic infection of immuno-compromised host and is responsible for high morbidity in hospitalized patients. Nosocomial infection with P. *aeruginosa* afflicts primarily patients submitted for prolonged treatment and receiving immuno-suppressive agents, corticosteroids, antimetabolites antibiotics or radiation.

The *Pseudomonas,* and particularly *P. aeruginosa,* produces a variety of toxins (such as hemolysins, fibrinolysins, esterases, coagulases, phospholipases, endo- and exo-toxins) that contribute to the pathogenicity of these bacteria. Moreover, these organisms have high intrinsic resistance to antibiotics due to the presence of multiple drug efflux pumps. This latter characteristic often complicates the outcome of the disease.

Due to the uncontrolled use of antibacterial chemotherapeutics the frequency of nosocomial infection caused by *P. aeruginosa* has increased considerably over the last 30 years. In the US, for example, the economic burden of *P. aeruginosa* nosocomial infection is estimated to 4.5 billion US$ annually. Therefore, the development of a vaccine for active or passive immunization against *P. aeruginosa* is actively needed (for review see Stanislavsky et al. 1997. FEMS Microbiol. Lett. 21: 243-277).

Various cell-associated and secreted antigens of *P. aeruginosa* have been the subject of vaccine development. Among *Pseudomonas* antigens, the mucoid substance, which is an extracellular slime consisting predominantly of alginate, was found to be heterogenous in terms of size and immunogenicity. High molecular mass alginate components (30-300 kDa) appear to contain conserved epitopes while lower molecular mass alginate components (10-30 kDa) possess conserved epitopes in addition to unique epitopes. Among surface-associated proteins, PcrV, which is part of the type III secretion-translocation apparatus, has also been shown to be an interesting target for vaccination (Sawa et al. 1999. Nature Medicine 5:392-398).

Surface-exposed antigens including O-antigens (O-specific polysaccharide of LPS) or H-antigens (flagellar antigens) have been used for serotyping due to their highly immunogenic nature. Chemical structures of repeating units of O-specific polysaccharides have been elucidated and these data allowed the identification of 31 chemotypes of *P. aeruginosa.* Conserved epitopes among all serotypes *of P. aeruginosa* are located in the core oligosaccharide and the lipid A region of LPS and immunogens containing these epitopes induce cross-protective immunity in mice against different *P. aemuginosa* immunotypes. The outer core of LPS was implicated to be a ligand for binding of *P. aeruginosa* to airway and ocular epithelial cells of animals. However, heterogeneity exists in this outer core region among different serotypes. Epitopes in the inner core are highly conserved and have been demonstrated to be surface-accessible, and not masked by O-specific polysaccharide.

To examine the protective properties of OM proteins, a vaccine containing *P*. *aeruginosa* OM proteins of molecular masses ranging from 20 to 100 kDa has been used in pre-clinical and clinical trials. This vaccine was efficacious in animal models against *P. aeruginosa* challenge and induced high levels of specific antibodies in human volunteers. Plasma from human volunteers containing anti-*P. aeruginosa* antibodies provided passive protection and helped the recovery of 87% of patients with severe forms of *P. aeruginosa* infection. More recently, a hybrid protein containing parts of the outer membrane proteins OprF (amino acids 190-342) and OprI (amino acids 21-83) *from Pseudomonas aeruginosa* fused to the glutathione-S-transferase was shown to protect mice against a 975-fold 50% lethal dose of *P*. *aeruginosa* (Knapp et al. 1999. Vaccine. 17:1663-1669).

However, the purification of blebs is technically difficult; bleb production in most Gram-negative strains results in poor yields of product for the industrial production of vaccines, and often in a very heterogeneous product. The present invention solves this problem by providing specially modified "hyperblebbing" strains from which blebs may be more easily made in higher yield and may be more homogeneous in nature. Such blebs may also be more readily filter sterilised.

In addition, if the bacteria make more blebs naturally, there are considerable process advantages associated with bleb purification in that blebs can be made and harvested without the use of detergents such as deoxycholate (for extraction of greater quantities of blebs). This would mean that usual process steps to remove detergent such as chromatography purification and ultra centrifugation may be obviated.

### BRIEF DESCRIPTION OF THE DRAWINGS

**Figure 1**: Multiple alignment of peptidoglycan-associated proteins. EC is *E. coli,* HI is *Haemophilus influenzae,* NG is *Neisseria gonorrhoeae.* ↑ indicates the position of the conserved F residue of OmpA homologues which should be conserved in C-terminal truncates. indicates the conserved full extent of the peptidoglycan-associating site.
**Figure 2**: Multiple alignment of peptidoglycan-associated proteins. EC is *E. coli,* MC is *Moraxella catarrhalis,* NG is *Neisseria gonorrhoeae.* ↑ indicates the position of the conserved F residue of OmpA homologues which should be conserved in C-terminal truncates. indicates the conserved full extent of the peptidoglycan-associating site.
**Figure 3**: Shows a hypothetical schematic structure of ompCD of M. catarrhalis. The location of the F residue of OmpA homologues which should be conserved in C-terminal truncates is shown, as is the peptidoglycan-associating site.
**Figure 4**: Shows PCR screening of recombinant Neisseria resulting from a double crossing over at the rmp locus as described in Example 1.
**Figure 5**: Schematic representation of the strategy used to construct the mutator plasmids for the deletion *of tol* genes in *Moraxella catarrhalis* and NTHI
**Figure 6**: A: Schematic representation of the expected double recombinant *tolQR Moraxella catarrhalis.* **B**: PCR analysis of recombinant *tol QR Moraxella catarrhalis* clones using primers E, F, G and H
**Figure 7**: Construction of the mutator plasmids used for the introduction of a stop codon into the *ompCD* sequence and *P5* sequence *of Moraxella catarrhalis* and NTHI respectively.

### DESCRIPTION OF THE INVENTION

In a first aspect, the present invention provides a hyperblebbing Gram-negative bacterium which has been genetically modified by either or both processes selected from a group consisting of: down-regulation of expression of one or more *tol* genes; and mutation of one or more gene(s) encoding a protein comprising a peptidoglycan-associated site to attenuate the peptidoglycan-binding activity of the protein(s).

By 'hyperblebbing' it is meant that the bacterium naturally sheds 2 times or more (more preferably 3, 4, 5, or 10 times or more) the quantity of blebs of the unmodified bacterium.

By 'down-regulation' and 'down-regulating' it is meant that expression of the gene in question is reduced (by at least 2 fold, preferably 5 fold or more) or switched off completely. This can readily be done by methods such as deleting the gene from the genome, introducing a stop codon into the coding sequence of the gene, deleting the promoter sequence of the gene, or replacing the promoter sequence of the gene for a weaker promoter. Where the gene is in an operon (as many *tol* genes are) care must be taken to ensure that the down-regulation of the target gene does not affect expression of the other genes in the operon that are not intended to be down regulated.

Specific *tol* genes may be identified in various Gram-negative bacteria by homology (preferably more than 20, 30, 40, 50, 60, 70, 80, 90% identity or more) to the *tol* genes described herein (for instance *tolA, B, Q* or *R),* or those of *E.coli.* Preferably 1, 2, 3, 4 or 5 *tol* genes are down-regulated in the bacterium of the invention. Most preferably pairs of *tol* genes: *tolQ* and *tolR,* or *tolR* and *tolA* are down-regulated (preferably by deletion or introduction of a disruptive stop codon) in a bacterium.

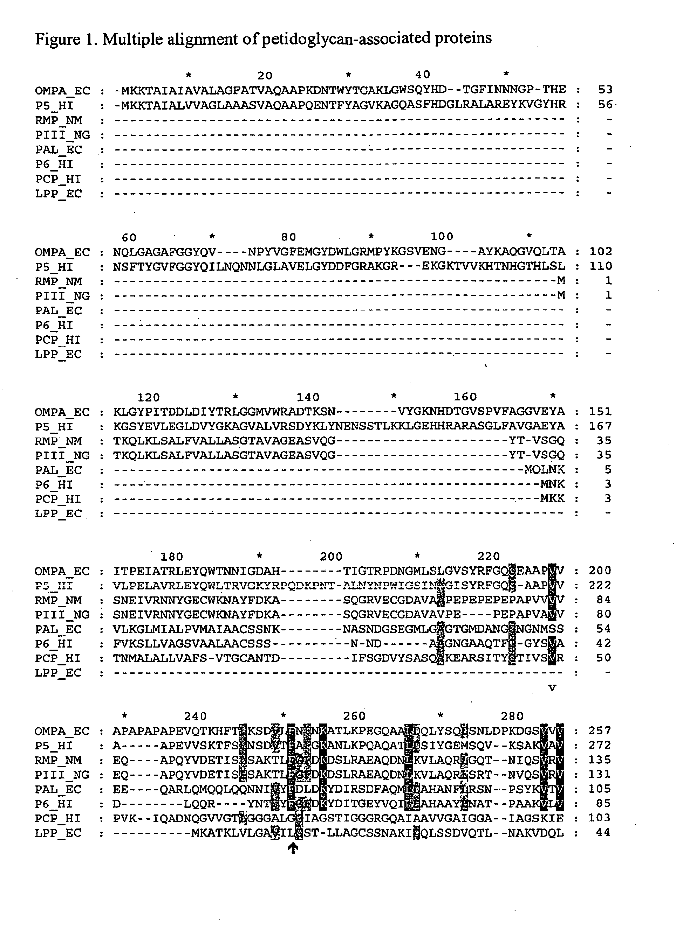

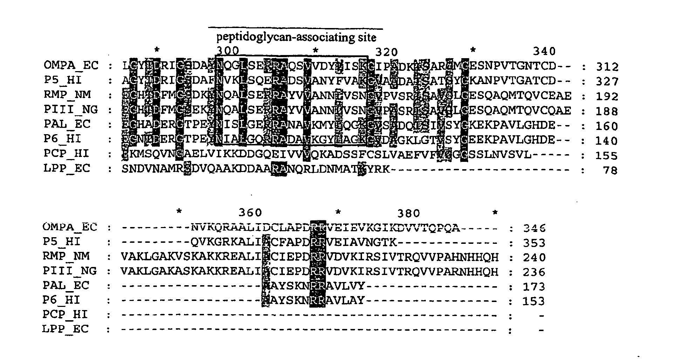

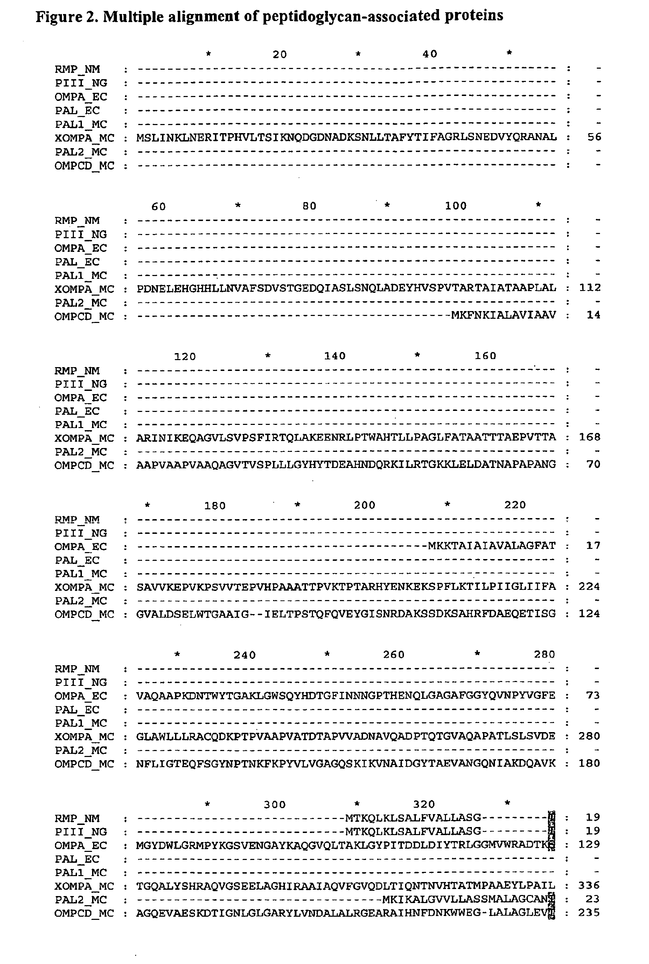

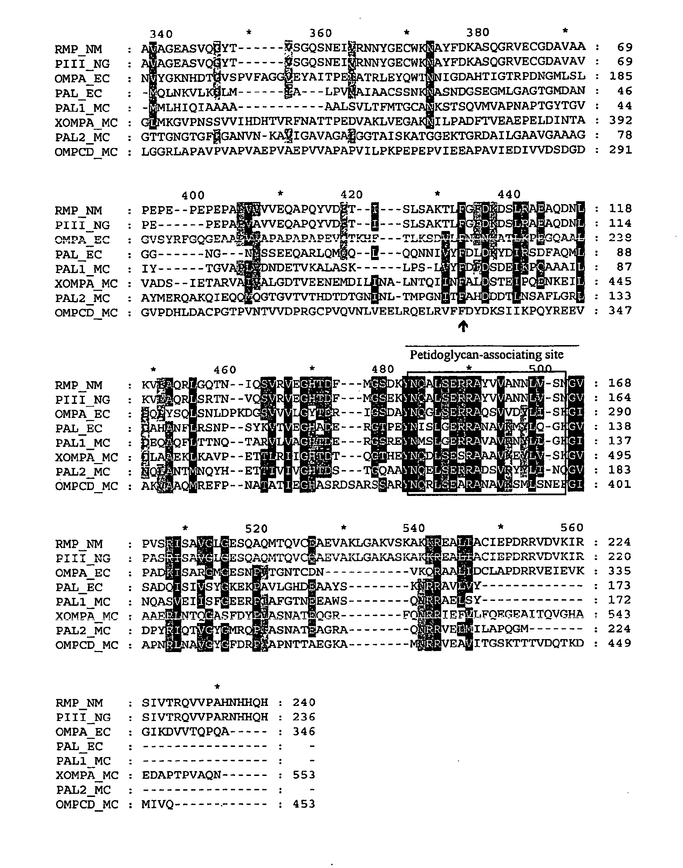

By 'mutation' of one or more gene(s) encoding a protein comprising a peptidoglycan-associated site to attenuate the peptidoglycan-binding activity of the protein(s) it is meant that such genes are either 'down-regulated' as described above. Alternatively, because such genes may encode protective antigens, a stop codon may be introduced within or 5' to the part of the gene encoding the peptidoglycan-associating site (a peptide of approximately 16-22 amino acids which is conserved and identifiable amongst Gram-negative bacterial strains, as shown in Fig. 1 and 2, or amino acid sequences 40, 50, 60, 70, 80, 90% or more identical to said sequences).

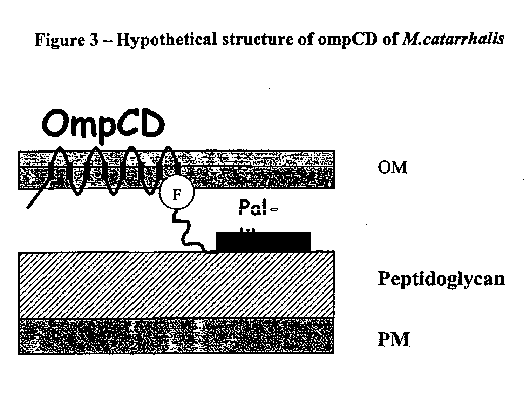

Frequently, such genes are integral membrane proteins, and therefore it is preferable for the stop codon to be 3' to the part of the gene encoding the outer-membrane associated part of the protein, and 5' to the peptidoglycan-associating site. It has been realised that for OmpA homologue proteins, such a stop codon should be placed 3' to a codon encoding a conserved F residue (as indicated in Fig. 1 and 2, and schematically in Fig. 3). This conserved F residue should be retained in order to ensure proper folding of the truncated protein in the outer membrane. C-terminal truncates of OmpA homologues (and genes encoding them) retaining this conserved F residue (the identity of which can readily be determined by comparison of a OmpA homologue to the sequence match-ups of Figs 1 and 2) is a further aspect of this invention.

When the region of the gene 3' of the region encoding the peptidoglycan-associating site is to be retained (for instance if it encodes a protective epitope [for instance in the case of P5 from *H. influenzae*]), the peptidoglycan-associating site may be engineered by 1, 2, 3, 4, 5 or more point mutations, or by deletion of amino acids (preferably 1, 2, 3, 4, 5, 7, 10, or 15 amino acids or the whole of the peptidoglycan-associating site) from the peptidoglycan-associating site, such that the peptidoglycan-binding activity of the protein is attenuated (reduced at least 2 fold, preferably removed entirely) to the desired level.

For the purposes of this invention 'peptidoglycan-associating site' means the region of a peptidoglycan-associating protein which can be aligned with the peptidoglycan-associating sites marked on Fig. 1 & 2 (either the boxed or delineated regions).

The above down-regulation and mutation events on the bacterial genome may be carried out by the skilled person using homologous recombination (as described in the Examples and in WO 01/09350 incorporated by reference herein). For this technique, knowledge of at least 50-100 nucleotides (preferably around 500) either side of the area of change should be known.

Bacteria harbouring mutations (e.g. knock-outs) of the *minB* locus are not intended to be covered by this invention, unless the bacterium has also been modified by either or both of the above processes of the invention.

The hyperblebbing Gram-negative bacterium may be selected from the group consisting of any bacterium from the *Neisseria* family (for instance *Neisseria meningitidis, Neisseria lactamica, Neisseria gonorrhoeae), Helicobacter pylori, Salmonella typhi, Salmonella typhimurium, Vibrio cholerae, Shigella spp., Haemophilus influenzae* (particularly non-typeable), *Bordetella pertussis, Pseudomonas aeruginosa* and *Moraxella catarrhalis.*

### Neisseria

In one embodiment the hyperblebbing Gram-negative bacterium is a *Neisseria* (preferably *Neisseria meningitidis)* strain which has been genetically modified by down-regulating expression of either or both of the following genes: *exbB* (homologous to *tolQ)* [SEQ ID NO:1] and *exbD* (homologous to *tolR)* [SEQ ID NO:3]. The upstream region of *exbB* and *exbD* is provided in SEQ ID NO:5 and 6, respectively, which is useful for designing homologous recombination vectors for down-regulating expression of the gene (for instance by deleting the promoter or replacing it with a weaker, or a metabolite-controlled promoter [e.g. the *phoE* promoter of *E.coli*])*.*

In a further embodiment the hyperblebbing *Neisseria* (preferably *Neisseria meningitidis)* strain has been genetically modified (in isolation or in combination with the above down-regulation events) by mutation of *rmpM* [SEQ ID NO:7 or 9] to attenuate the peptidoglycan-binding activity of the encoded protein. The peptidoglycan-associating site for the protein can be seen in Fig. 1 (and has the amino acid sequence NQALSERRAYVVANNLVSN - see also SEQ ID NO:8). The upstream region of the gene is provided in SEQ ID NO:10 which is useful for the down-regulation of the gene. Preferably the gene is mutated in the way described in Example 1. If a truncate is made, it is preferred to introduce the stop codon downstream of the codon encoding the conserved F residue as indicated in Fig. 1 and 2.

Vesicles prepared from such modifed strains may have one or more of the following improvements: reduced particle size (allowing sterile filtration through 0.22 µm pores), an increased batch homogeneity, and a superior yield. Such kind of alterations on bleb morphology are obtained by manipulating genes involved in linking the outer membrane to the peptidoglycan layer and/or to the cytoplasmic membrane as described above. Improved, natural bleb shedding has the advantage that blebs may be isolated in industrial quantities without the use of detergents such as deoxycholate.

### Haemophilus influenzae

In one embodiment the hyperblebbing Gram-negative bacterium is a *Haemophilus influenzae* (preferably non-typeable) strain which has been genetically modified by down-regulating expression of one or more of the following genes: *tolQ* [SEQ ID NO:11], *tolR* [SEQ ID NO:13], *tolA* [SEQ ID NO:15] and *tolB* [SEQ ID NO:17]. The genes are present in a single operon, and thus the upstream region provided in SEQ ID NO:19, is useful for designing homologous recombination vectors for down-regulating expression of all genes on the operon (for instance by deleting the promoter or replacing it with a weaker, or a metabolite controlled promoter [e.g. the *phoE* promoter of *E.coli*]). Preferred embodiments include deleting both *tolQ & R* genes, or both *tolR & A* genes (preferably as described in Examples 4 and 5, respectively), whilst maintaining expression of the other genes on the operon (particularly *tolB*).

In a further embodiment the hyperblebbing *Haemophilus influenzae* (preferably non-typeable) strain has been genetically modified (in isolation or in combination with the above down-regulation events) by mutation of of one or more genes selected from a group consisting of: *ompP5* [SEQ ID NO:20], *ompP6* [SEQ ID NO:22 or 24] and *pcp* [SEQ ID NO:26] to attenuate the peptidoglycan-binding activity of the encoded protein. The peptidoglycan-associating site for the proteins can be seen in Fig. 1. Preferably the genes are mutated in a similar way to that described in Example 6. If a truncate is made of P5 or P6, it is preferred to introduce the stop codon downstream of the codon encoding the conserved F residue as indicated in Fig. 1.

For P5, the region of the gene 3' of the region encoding the peptidoglycan-associating site may advantageously be retained (as it encodes a protective epitope). In such case, the peptidoglycan-associating site may be engineered by 1, 2, 3, 4, 5 or more point mutations, or by deletion of amino acids (preferably 1, 2, 3, 4, 5, 7, 10, or 15 amino acids, or the whole of the peptidoglycan-associating site) from the peptidoglycan-associating site, such that the peptidoglycan-binding activity of the protein is reduced (preferably, removed entirely) to the desired level, whilst retaining the protective epitope.

Preferred bacteria have down-regulated *tolQ&R* and mutated *P5,* or down-regulated *tolR&A* and mutated *P5* phenotypes.

The P5 gene has been found to be homologous with *E. coli* OmpA gene, and the P6 gene has been found to be homologous with *E. coli* Pal gene (P5 and OmpA proteins are 51 % identical, P6 and Pal proteins are 62% identical). The pcp gene (also called Ipp) encodes a lipoprotein similar neither *to E coli* Lpp nor to *E coli* Pal, but contains a peptidoglycan-associating site (Fig. 1).

Vesicles prepared from such modified strains may have one or more of the following improvements: reduced particle size (allowing sterile filtration through 0.22 µm pores), an increased batch homogeneity, and a superior yield. Such kind of alterations on bleb morphology are obtained by manipulating genes involved in linking the outer membrane to the peptidoglycan layer and/or to the cytoplasmic membrane as described above. Improved, natural bleb shedding has the advantage that blebs may be isolated in industrial quantities without the use of detergents such as deoxycholate.

### Moraxella catarrhalis

In one embodiment the hyperblebbing Gram-negative bacterium is a *Moraxella catarrhalis* strain which has been genetically modified by down-regulating expression of one or more of the following genes: *tolQ* [SEQ ID NO:28], *tolR* [SEQ ID NO:30], *tolX* [SEQ ID NO:32], *tolB* [SEQ ID NO:34] and *tolA* [SEQ ID NO:36]. The tolQRXB genes are present in a single operon, and thus the upstream region provided upstream of SEQ ID NO:28, is useful for designing homologous recombination vectors for down-regulating expression of all genes on the operon (for instance by deleting the promoter or replacing it with a weaker, or a metabolite-controlled promoter [e.g. the *phoE* promoter of *E.coli]).* Upstream sequence is also provided upstream of SEQ ID NO:36 for similarly doing so to the *tolA* gene. Preferred embodiments include deleting both *tolQ & R* genes, or both *tolR & X* genes (preferably as described in Example 2), whilst maintaining expression of the other genes on the operon (particularly *tolB*).

In a further embodiment the hyperblebbing *Moraxella catarrhalis* strain has been genetically modified (in isolation or in combination with the above down-regulation events) by mutation of of one or more genes selected from a group consisting of: *ompCD* [SEQ ID NO:38], *xompA* [SEQ ID NO:40; WO 00/71724], *pall* [SEQ ID NO:42], and *pal2* [SEQ ID NO:44], to attenuate the peptidoglycan-binding activity of the encoded protein. The peptidoglycan-associating site for the proteins can be seen in Fig. 2. Preferably the genes are mutated in a similar way to that described in Example 3. If a truncate is made of OMPCD, XOMPA or Pall or Pa12, it is preferred to introduce the stop codon downstream of the codon encoding the conserved F residue as indicated in Fig. 2.

Preferred bacteria have down-regulated *tolQ&R* and mutated *ompCD,* or down-regulated *tolR&X* and mutated *ompCD* phenotypes.

The OMPCD gene has been found to be homologous with *E. coli* OmpA gene. The OmpCD encoded protein is not well conserved in its N-terminal domain, compared to OmpA. However, it contains a proline, alanine and valine rich "hinge" region and its C-terminal domain is significantly similar to the C-terminal domain of OmpA (25% identity in 147 aa overlap). Two genes encoding lipoproteins related to Pal have also been identified (Pall and Pa12 are respectivily 39% and 28% identical to *E. coli* Pal). These lipoproteins, as well as the C-terminal domain of OmpCD, contain a putative PgAS (Fig. 2). A fourth gene (xOmpA) encoding a protein containing a putative PgAS has been identified in *M catarrhalis.* The N-terminal domain of this protein shows no significant similarity to any known protein. However, its C-terminal domain is similar to the C-terminal domain of OmpA (25% identity in 165 aa overlap) (Figure 2).

Vesicles prepared from such modifed strains may have one or more of the following improvements: reduced particle size (allowing sterile filtration through 0.22 µm pores), an increased batch homogeneity, and a superior yield. Such kind of alterations on bleb morphology are obtained by manipulating genes involved in linking the outer membrane to the peptidoglycan layer and/or to the cytoplasmic membrane as described above. Improved, natural bleb shedding has the advantage that blebs may be isolated in industrial quantities without the use of detergents such as deoxycholate.

### Further improvements in the bacteria and blebs of the invention

The hyperblebbing Gram-negative bacterium may be further genetically engineered by one or more processes selected from the following group: (a) a process of down-regulating expression of immunodominant variable or non-protective antigens, (b) a process of upregulating expression of protective OMP antigens, (c) a process of down-regulating a gene involved in rendering the lipid A portion of LPS toxic, (d) a process of upregulating a gene involved in rendering the lipid A portion of LPS less toxic, and (e) a process of down-regulating synthesis of an antigen which shares a structural similarity with a human structure and may be capable of inducing an auto-immune response in humans.

Such bleb vaccines of the invention are designed to focus the immune response on a few protective (preferably conserved) antigens or epitopes - formulated in a multiple component vaccine. Where such antigens are integral OMPs, the outer membrane vesicles of bleb vaccines will ensure their proper folding. This invention provides methods to optimize the OMP and LPS composition of OMV (bleb) vaccines by deleting immunodominant variable as well as non protective OMPs, by creating conserved OMPs by deletion of variable regions, by upregulating expression of protective OMPs, and by eliminating control mechanisms for expression (such as iron restriction) of protective OMPs. In addition the invention provides for the reduction in toxicity of lipid A by modification of the lipid portion or by changing the phosphoryl composition whilst retaining its adjuvant activity or by masking it. Each of these new methods of improvement individually improve the bleb vaccine, however a combination of one or more of these methods work in conjunction so as to produce an optimised engineered bleb vaccine which is immuno-protective and non-toxic - particularly suitable for paediatric use.

### (a) a process of down-regulating expression of immunodominant variable or non-protective antigens

Many surface antigens are variable among bacterial strains and as a consequence are protective only against a limited set of closely related strains. An aspect of this invention covers the reduction in expression, or, preferably, the deletion of the gene(s) encoding variable surface protein(s) which results in a bacterial strain producing blebs which, when administered in a vaccine, have a stronger potential for cross-reactivity against various strains due to a higher influence exerted by conserved proteins (retained on the outer membranes) on the vaccinee's immune system. Examples of such variable antigens include: for *Neisseria* - pili (PilC) which undergoes antigenic variations, PorA, Opa, TbpB, FrpB; for *H. influenzae* - P2, P5, pilin, IgA1-protease; and for *Moraxella* - CopB, OMP106.

Other types of gene that could be down-regulated or switched off are genes which, *in vivo,* can easily be switched on (expressed) or off by the bacterium. As outer membrane proteins encoded by such genes are not always present on the bacteria, the presence of such proteins in the bleb preparations can also be detrimental to the effectiveness of the vaccine for the reasons stated above. A preferred example to down-regulate or delete is *Neisseria* Opc protein. Anti-Opc immunity induced by an Opc containing bleb vaccine would only have limited protective capacity as the infecting organism could easily become Opc⁻. *H. influenzae* HgpA and HgpB are other examples of such proteins.

In process a), these variable or non-protective genes are down-regulated in expression, or terminally switched off. This has the surprising advantage of concentrating the immune system on better antigens that are present in low amounts on the outer surface of blebs.

The strain can be engineered in this way by a number of strategies including transposon insertion to disrupt the coding region or promoter region of the gene, or point mutations or deletions to achieve a similar result. Homologous recombination may also be used to delete a gene from a chromosome (where sequence X comprises part (preferably all) of the coding sequence of the gene of interest). It may additionally be used to change its strong promoter for a weaker (or no) promoter. All these techniques are described in WO 01/09350 (published by WIPO on 8/2/01 and incorporated by reference herein).

### (b) a process of upregulating expression of protective OMP antigens

This may be done by inserting a copy of such a protective OMP into the genome (preferably by homologous recombination), or by upregulating expression of the native gene by replacing the native promoter for a stronger promoter, or inserting a strong promoter upstream of the gene in question (also by homologous recombination). Such methods can be accomplished using the techniques described in WO 01/09350 (published by WIPO on 8/2/01 and incorporated by reference herein).

Such methods are particularly useful for enhancing the production of immunologically relevant Bleb components such as outer-membrane proteins and lipoproteins (preferably conserved OMPs, usually present in blebs at low concentrations).

### (c) a process of down-regulating a gene involved in rendering the lipid A portion of LPS toxic

The toxicity of bleb vaccines presents one of the largest problems in the use of blebs in vaccines. A further aspect of the invention relates to methods of genetically detoxifying the LPS present in Blebs. Lipid A is the primary component of LPS responsible for cell activation. Many mutations in genes involved in this pathway lead to essential phenotypes. However, mutations in the genes responsible for the terminal modifications steps lead to temperature-sensitive (*htrB*) or permissive *(msbB)* phenotypes. Mutations resulting in a decreased (or no) expression of these genes result in altered toxic activity of lipid A. Indeed, the non-lauroylated (htrB mutant) [also defined by the resulting LPS lacking both secondary acyl chains] or non-myristoylated (msbB mutant) [also defined by the resulting LPS lacking only a single secondary acyl chain] lipid A are less toxic than the wild-type lipid A. Mutations in the lipid A 4'-kinase encoding gene (*lpxK* also decreases the toxic activity of lipid A.

Process c) thus involves either the deletion of part (or preferably all) of one or more of the above open reading frames or promoters. Alternatively, the promoters could be replaced with weaker promoters. Preferably the homologous recombination techniques are used to carry out the process. Preferably the methods described in WO 01/09350 (published by WIPO on 8/2/01 and incorporated by reference herein) are used. The sequences of the htrB and msbB genes from *Neisseria meningitidis B, Moraxella catarrhalis,* and *Haemophilus influenzae* are provided in WO 01/09350 for this purpose.

### (d) a process of upregulating a gene involved in rendering the lipid A portion of LPS less toxic .

LPS toxic activity could also be altered by introducing mutations in genes/loci involved in polymyxin B resistance (such resistance has been correlated with addition of aminoarabinose on the 4' phosphate of lipid A). These genes/loci could be *pmrE* that encodes a UDP-glucose dehydrogenase, or a region of antimicrobial peptide-resistance genes common to many enterobacteriaciae which could be involved in aminoarabinose synthesis and transfer. The gene *pmrF* that is present in this region encodes a dolicol-phosphate manosyl transferase (Gunn J.S., Kheng, B.L., Krueger J., Kim K.,Guo L., Hackett M., Miller S.I. 1998. Mol. Microbiol. 27: 1171-1182).

Mutations in the PhoP-PhoQ regulatory system, which is a phospho-relay two component regulatory system (f. i. PhoP constitutive phenotype, PhoP^{c}), or low Mg⁺⁺ environmental or culture conditions (that activate the PhoP-PhoQ regulatory system) lead to the addition of aminoarabinose on the 4'-phosphate and 2-hydroxymyristate replacing myristate (hydroxylation of myristate). This modified lipid A displays reduced ability to stimulate E-selectin expression by human endothelial cells and TNF-α secretion from human monocytes.

Process d) involves the upregulation of these genes using a strategy as described in WO 01/09350 (published by WIPO on 8/2/01 and incorporated by reference herein).

### (e) a process of down-regulating synthesis of an antigen which shares a structural similarity with a human structure and may be capable of inducing an auto-immune response in humans

The isolation of bacterial outer-membrane blebs from encapsulated Gram-negative bacteria often results in the co-purification of capsular polysaccharide. In some cases, this "contaminant" material may prove useful since polysaccharide may enhance the immune response conferred by other bleb components. In other cases however, the presence of contaminating polysaccharide material in bacterial bleb preparations may prove detrimental to the use of the blebs in a vaccine. For instance, it has been shown at least in the case of *N. meningitidis* that the serogroup B capsular polysaccharide does not confer protective immunity and is susceptible to induce an adverse auto-immune response in humans. Consequently, process e) of the invention is the engineering of the bacterial strain for bleb production such that it is free of capsular polysaccharide. The blebs will then be suitable for use in humans. A particularly preferred example of such a bleb preparation is one from *N. meningitidis* serogroup B devoid of capsular polysaccharide.

This may be achieved by using modified bleb production strains in which the genes necessary for capsular biosynthesis and/or export have been impaired as described in WO 01/09350 (published by WIPO on 8/2/01 and incorporated by reference herein). A preferred method is the deletion of some or all of the *Neisseria meningitidis cps* genes required for polysaccharide biosynthesis and export. For this purpose, the replacement plasmid pMF121 (described in Frosh et al.1990, Mol. Microbiol. 4:1215-1218) can be used to deliver a mutation deleting the *cpsCAD* (+ *galE*) gene cluster. Alternatively the *siaD* gene could be deleted, or down-regulated in expression (the meningococcal *siaD* gene encodes alpha-2,3-sialyltransferase, an enzyme required for capsular polysaccharide and LOS synthesis). Such mutations may also remove host-similar structures on the saccharide portion of the LPS of the bacteria.

### Combinations of methods a) - e)

It may be appreciated that one or more of the above processes may be used to produce a modified strain from which to make improved bleb preparations of the invention. Preferably one such process is used, more preferably two or more (2, 3, 4, or 5) of the processes are used in order to manufacture the bleb vaccine. As each additional method is used in the manufacture of the bleb vaccine, each improvement works in conjunction with the other methods used in order to make an optimised engineered bleb preparation.

A preferred meningococcal (particularly *N. meningitidis* B) bleb preparation comprises the use of processes b), c) and e) (optionally combined with process a)). Such bleb preparations are safe (no structures similar to host structures), non-toxic, and structured such that the host immune response will be focused on high levels of protective (and preferably conserved) antigens. All the above elements work together in order to provide an optimised bleb vaccine.

Similarly for *M. catarrhalis,* non-typeable *H. influenzae,* and non serotype B meningococcal strains (e.g. serotype A, C, Y or W), preferred bleb preparations comprise the use of processes b) and c), optionally combined with process a).

### Preferred Neisserial bleb preparations

One or more of the following genes (encoding protective antigens) are preferred for upregulation via process b) when carried out on a Neisserial strain, including gonococcus, and meningococcus (particularly *N. meningitidis* B): NspA (WO 96/29412), Hsf-like (WO 99/31132), Hap (PCT/EP99/02766), PorA, PorB, OMP85 (WO 00/23595), PilQ (PCT/EP99/03603), PldA (PCT/EP99/06718), FrpB (WO 96/31618), TbpA (US 5,912,336), TbpB, FrpA/FrpC (WO 92/01460), LbpA/LbpB (PCT/EP988/05117), FhaB (WO 98/02547), HasR (PCT/EP99/05989), lipo02 (PCT/BP99/08315), Tbp2 (WO 99/57280), MltA (WO 99/57280), and ctrA (PCT/EP00/00135). They are also preferred as genes which may be heterologously introduced into other Gram-negative bacteria.

One or more of the following genes are preferred for downregulation via process a): PorA, PorB, PilC, TbpA, TbpB, LbpA, LbpB, Opa, and Opc (most preferably PorA).

One or more of the following genes are preferred for downregulation via process c): htrB, msbB and lpxk (most preferably msbB which removes only a single secondary acyl chain from the LPS molecule).

One or more of the following genes are preferred for upregulation via process d): pmrA, pmrB, pmrE, and pmrF.

One or more of the following genes are preferred for downregulation via process e): galE, siaA, siaB, siac, siaD, ctrA, ctrB, ctrC, and ctrD (the genes are described in described in WO 01/09350 - published by WIPO on 8/2/01 and incorporated by reference herein).

### Preferred Pseudomonas aeruginosa bleb preparations

One or more of the following genes (encoding protective antigens) are preferred for upregulation via process b): PcrV, OprF, OprI. They are also preferred as genes which may be heterologously introduced into other Gram-negative bacteria.

### Preferred Moraxella catarrhalis bleb preparations

One or more of the following genes (encoding protective antigens) are preferred for upregulation via process b): OMP106 (WO 97/41731 & WO 96/34960), HasR (PCT/EP99/03824), PilQ (PCT/EP99/03823), OMP85 (PCT/EP00/01468), lipo06 (GB 9917977.2), lipo10 (GB 9918208.1), lipo11 (GB 9918302.2), lipo18 (GB 9918038.2), P6 (PCT/EP99/03038), ompCD, CopB (Helminen ME, et al (1993) Infect. Immun. 61:2003-2010), D15 (PCT/EP99/03822), OmplA1 (PCT/EP99/06781), Hly3 (PCT/EP99/03257), LbpA and LbpB (WO 98/55606), TbpA and TbpB (WO 97/13785 & WO 97/32980), OmpE, UspA1 and UspA2 (WO 93/03761), FhaB (WO 99/58685) and Omp21. They are also preferred as genes which may be heterologously introduced into other Gram-negative bacteria.

One or more of the following genes are preferred for downregulation via process a): CopB, OMP106, OmpB1, TbpA, TbpB, LbpA, and LbpB.

One or more of the following genes are preferred for downregulation via process c): htrB, msbB and lpxK (most preferably msbB).

One or more of the following genes are preferred for upregulation via process d): pmrA, pmrB, pmrE, and pmrF.

### Preferred Haemophilus influenzae bleb preparations

One or more of the following genes (encoding protective antigens) are preferred for upregulation via process b): D15 (WO 94/12641), P6 (EP 281673), TbpA, TbpB, P2, P5 (WO 94/26304), OMP26 (WO 97/01638), HMW1, HMW2, HMW3, HMW4, Hia, Hsf, Hap, Hin47, Iomp1457 (GB 0025493.8), YtfN (GB 0025488.8), VirG (GB 0026002.6), Iomp1681 (GB 0025998.6), OstA (GB 0025486.2) and Hif (all genes in this operon should be upregulated in order to upregulate pilin). They are also preferred as genes which may be heterologously introduced into other Gram-negative bacteria.

One or more of the following genes are preferred for downregulation via process a): P2, P5, Hif, IgA1-protease, HgpA, HgpB, HMW1, HMW2, Hxu, TbpA, and TbpB.

One or more of the following genes are preferred for downregulation via process c): htrB, msbB and lpxK (most preferably msbB).

One or more of the following genes are preferred for upregulation via process d): pmrA, pmrB, pmrE, and pmrF.

### Preparations of membrane vesicles (blebs) of the invention

The manufacture of bleb preparations from any of the aforementioned modified strains may be achieved by harvesting blebs naturally shed by the bacteria, or by any of the methods well known to a skilled person (e.g. as disclosed in EP 301992, US 5,597,572, EP 11243 or US 4,271,147).

A preparation of membrane vesicles obtained from the bacterium of the invention is a further aspect of this invention. Preferably, the preparation of membrane vesicles is capable of being filtered through a 0.22 µm membrane.

A sterile (preferably homogeneous) preparation of membrane vesicles obtainable by passing the membrane vesicles from the bacterium of the invention through a 0.22 µm membrane is also envisaged.

### Vaccine Formulations

A vaccine which comprises a bacterium of the invention or a bleb preparation of the invention together with a pharmaceutically acceptable diluent or carrier is a further aspect of the invention. Such vaccines are advantageously used in a method of treatment of the human or animal body.

Vaccine preparation is generally described in Vaccine Design ("The subunit and adjuvant approach" (eds Powell M.F. & Newman M.J.) (1995) Plenum Press New York).

The vaccine preparations of the present invention may be adjuvanted. Suitable adjuvants include an aluminium salt such as aluminum hydroxide gel (alum) or aluminium phosphate, but may also be a salt of calcium (particularly calcium carbonate), iron or zinc, or may be an insoluble suspension of acylated tyrosine, or acylated sugars, cationically or anionically derivatised polysaccharides, or polyphosphazenes.

Suitable Th1 adjuvant systems that may be used include, Monophosphoryl lipid A, particularly 3-de-O-acylated monophosphoryl lipid A, and a combination of monophosphoryl lipid A, preferably 3-de-O-acylated monophosphoryl lipid A (3D-MPL) together with an aluminium salt. An enhanced system involves the combination of a monophosphoryl lipid A and a saponin derivative particularly the combination of QS21 and 3D-MPL as disclosed in WO 94/00153, or a less reactogenic composition where the QS21 is quenched with cholesterol as disclosed in WO96/33739. A particularly potent adjuvant formulation involving QS21 3D-MPL and tocopherol in an oil in water emulsion is described in WO95/17210 and is a preferred formulation.

The vaccine may comprise a saponin, more preferably QS21. It may also comprise an oil in water emulsion and tocopherol. Unmethylated CpG containing oligo nucleotides (WO 96/02555) are also preferential inducers of a TH1 response and are suitable for use in the present invention.

The vaccine preparation of the present invention may be used to protect or treat a mammal susceptible to infection, by means of administering said vaccine via systemic or mucosal route. These administrations may include injection *via* the intramuscular, intraperitoneal, intradermal or subcutaneous routes; or *via* mucosal administration to the oral/alimentary, respiratory, genitourinary tracts. Thus one aspect of the present invention is a method of protecting an individual against a bacterial infection which comprises administering to the individual an effective amount (capable of immunoprotecting an individual against the source bacterium) of a bacterium of the invention or a bleb preparation of the invention.

The amount of antigen in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-100µg of protein antigen, preferably 5-50µg, and most typically in the range 5 - 25µg.

An optimal amount for a particular vaccine can be ascertained by standard studies involving observation of appropriate immune responses in subjects. Following an initial vaccination, subjects may receive one or several booster immunisations adequately spaced.

A process for preparing a vaccine composition comprising a preparation of membrane vesicles of the invention is also envisaged which process comprises: (a) inoculating a culture vessel containing a nutrient medium suitable for growth of the bacterium of the invention; (b) culturing said bacterium; (c) recovering membrane vesicles from the medium; and (d) mixing said membrane vesicles with a pharmaceutically acceptable diluent or carrier. The vesicles may be recovered by detergent (e.g. deoxycholate) extraction, but are preferably recovered without such a step (and necessary chromatography and ultracentrifugation steps that go with it)

Preferably after either step (c) or step (d), the prepartion is sterile-filtered (through a 0.22 µm membrane).

A method for producing a hyperblebbing bacterium or the invention is also provided, which method comprises genetically modifying a Gram-negative bacterial strain by either or both of the following processes: (a) engineering the strain to down-regulate expression of one or more *Tol* genes; and (b) mutating one or more gene(s) encoding a protein comprising a peptidoglycan-associated site to attenuate the peptidoglycan-binding activity of the protein(s).

### Nucleotide sequences of the invention

A further aspect of the invention relates to the provision of nucleotide sequences (see appended sequence listings) which may be used in the processes (down-regulation/mutation) of the invention.

Another aspect of the invention is an isolated polynucleotide sequence which hybridises under highly stringent conditions to at least a 30 nucleotide portion of a nucleotide sequence of the invention (e.g. SEQ ID NO:1, 3, 5, 6, 7, 9, 10, 11, 13, 15, 17, 19, 20, 22, 24, 26, 28, 30, 32, 34, 36, 38, 40, 42, or 44) or a complementary strand thereof. Preferably the isolated sequence should be long enough to perform homologous recombination with the chromosomal sequence if it is part of a suitable vector - namely at least 30 nucleotides (preferably at least 40, 50, 60, 70, 80, 90, 100, 200, 300, 400, or 500 nucleotides). More preferably the isolated polynucleotide should comprise at least 30 nucleotides (preferably at least 40, 50, 60, 70, 80, 90, 100, 200, 300, 400, or 500 nucleotides) of the actual sequences provided or a complementary strand thereof.

As used herein, highly stringent hybridization conditions include, for example, 6X SSC, 5X Denhardt, 0.5% SDS, and 100 µg/mL fragmented and denatured salmon sperm DNA hybridized overnight at 65 °C and washed in 2X SSC, 0.1% SDS one time at room temperature for about 10 minutes followed by one time at 65 °C for about 15 minutes followed by at least one wash in 0.2X SCC, 0.1 % SDS at room temperature for at least 3-5 minutes.

A further aspect is the use of the isolated polynucleotide sequences of the invention in performing a genetic engineering event (such as transposon insertion, or site specific mutation or deletion, but preferably a homologous recombination event) within a Gram-negative bacterial chromosomal gene in order to down-regulate or mutate it as described above. Preferably the strain in which the recombination event is to take place is the same as the strain from which the sequences of the invention were obtained. However, the meningococcus A, B, C, Y and W and gonococcus genomes are sufficiently similar that sequence from any of these strains may be suitable for designing vectors for performing such events in the other strains. This is likely also to be the case for *Haemophilus influenzae* and non-typeable *Haemophilus influenzae.*

Cited documents are incorporated by reference herein.

### EXAMPLES

The examples below are carried out using standard techniques, which are well known and routine to those of skill in the art, except where otherwise described in detail. The examples are illustrative, but do not limit the invention.

### Example 1:

### Construction of a Neisseria meningitidis strain lacking functional RmpM gene

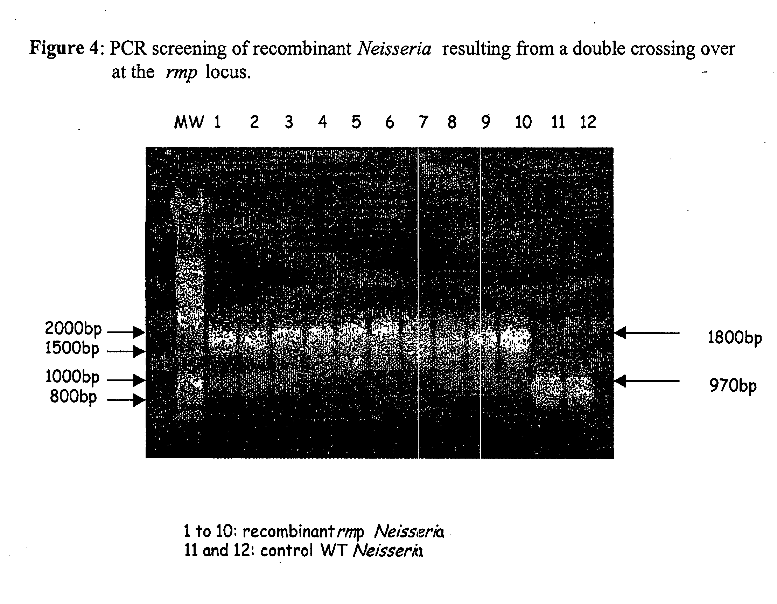

The aim of the experiment was to construct a *Neisseria meningitidis* serogroup B strain expressing a truncated Rmp protein. *Neisseria meningitidis* Rmp is homologous to *E. coli* OmpA and *P. aeruginosa* OprF. This protein contains an N-terminal domain anchored in the external membrane, and a C-terminal domain containing a peptidoglycan associated site. The C-terminal domain of Rmp was deleted by homologous recombination in a *Neisseria meningitidis* serogroup B *cps-* strain. The expressed N-terminal part of the protein will still play its role in the stability of the external membrane, while the absence of the peptidoglycan associated site will relax the membrane around the bacterium. Outer membrane vesicles from this modified *Neisseria* were analyzed: amount of production, size, homogeneity. A DNA region (729bp) corresponding to the *rmp* gene was discovered (SEQID N° 9) in the Sanger database containing genomic DNA sequences of the *Neisseria meningitidis* serogroup A strain Z2491. A similar sequence is present in *Neisseria meningitidis* serogroup B strain MC58 (SEQID N° 7); it shows 99.3% identity with the men A sequence. A DNA fragment covering the complete sequence of the gene was PCR amplified from *Neisseria meningitidis* serogroup B genomic DNA, using oligonucleotides **RMP-H-5** (5'- GCC CAC AAG CTT ATG ACC AAA CAG CTG AAA TT-3') & **RMP-E-3** (5'- CCG GAA TTC TTA GTG TTG GTG ATG ATT GT-3') containing *Hind*III and *EcoR*I restriction sites (underlined). This PCR fragment was cleaned with a High Pure Kit (Roche, Mannheim, Germany) and directly cloned in a pGemT vector (Promega, USA). This plasmid was submitted to circle PCR mutagenesis (Jones & Winistofer (1992), Biotechniques 12: 528-534) in order to introduce a 33bp deletion and a stop codon after the internal phenylalanine residue. The circle PCR was performed using the oligonucleotides **RMP-CIRC-3-B** (5'-GGC GGA TCC TTA GAA CAG GGT TTT GGC AG-3') & **RMP CIRC-5-B** (5'-CGG GGA TCC CAA GAC AAC CTG AAA GTA TT-3') containing *BamH*I restriction sites (underlined). The *cmR* gene was amplified from pGPS2 plasmid, with oligonucleotides **CM/BAM/5/2** (5'-CGC GGA TCC GCC GTC TGA AAC CTG TGA CGG AAG ATC AC-3') & **CM/BAM/3/2** (5'-CGC GGA TCC TTC AGA CGG CCC AGG CGT TTA AGG GCA C-3') containing uptake sequences and *BamH*I restriction sites (underlined). This fragment was inserted in the circle PCR plasmid restricted with *BamH*I. The recombinant plasmid was used to transform *Neisseria meningitidis* serogrouo B *cps-,* strain. Recombinant *Neisseria meningitidis* resulting from a double crossing over event were selected by PCR screening with primers **RMP SCR 5** (5'- CAT GAT AGA CTA TCA GGA AAC-3') and **RMP SCR 3** (5'-CAG TAC CTG GTACAA AAT CC-3'). Those primers amplify a fragment of 970bp from the control strain (WT for *rmp*) and one of 1800bp from the recombinant *Neisseria.* Fig. 4 shows the PCR amplifications obtained from 10 recombinant colonies analyzed on a 1% agarose gel in the presence of ethidium bromide. Recombinants were grown on GC medium containing 5µg/ml chloramphenicol and analyzed for Rmp expression and OMV production.

### Characterization of menB OMV's produced from an rmpM mutant

The effect of the rmpM mutation on OMV's yield, size and polydispersity was analyzed by comparing OMV's extracted (using Deoxycholate) from parental H44/76 Cps- (no capsular polysaccharide) and the corresponding OMV's extracted from the RmpM mutant derivative. The results are the following:

| **OMV's yields observed with different *N. meningitidis* H44/76 derivative strains grown in 400 ml Flask cultures** |
|---|
| Strain Nm B1390 cps(-) *porA*(+) PilQ atg : 2.7 mg |
| Strain Nm B1391 cps(-) *porA*(-) PilQ atg : 9.1 mg |
| Strain B1405 cps(-) *porA*(-) RmpM (-) : 20 mg |

As shown below, deletion of *rmpM* significantly increase (at least a factor 2) the yield of OMV's prepared from such a strain. The size of OMV's isolated from wild-type and *rmpM* mutants *N. meningitidis* H44/46 derivative strains was estimated by Photon Correlation Spectroscopy (PCS) using the Malvern Zetasizer 4000 analyzer as recommended by the supplier (Malvern Instruments GmbH, Herrenberg Germany : www.malvern.co.uk). Results are summarized below:

| Samples | Z average diameter (nm) | Polydispersity |
|---|---|---|
| CPS (-) 07/2000 7.8mg/ml | 136 | 0.31 |
| CPS (-) 09/2000 5.7mg/ml | 166 | 0.42 |
| CPS (-)*rmpM* (-) B1405 6.7mg/ml | 202 | 0.53 |

These data support that the size of CPS (-) 07/2000 is smaller than the size of CPS (-) 09/2000 and also that the size of CPS (-) samples is smaller than the size of CPS (-) *rmpM* (-) blebs. Altogether, these data support that deletion of a domain encoding the peptidoglycan associated domain of RmpM leads to enhanced blebbing and altered OMV morphology and size distribution. These features could be advantageously used for the production of vaccines as documented in WO 01/09350 (published by WIPO on 8/2/01 and incorporated by reference herein).

### Example 2. Deletion of the tolQR genes in Moraxella catarrhalis

The aim of the experiment was to delete the *tolQR* genes from *Moraxella catarrhalis* in order to obtain a hyperblebbing *Moraxella* strain.

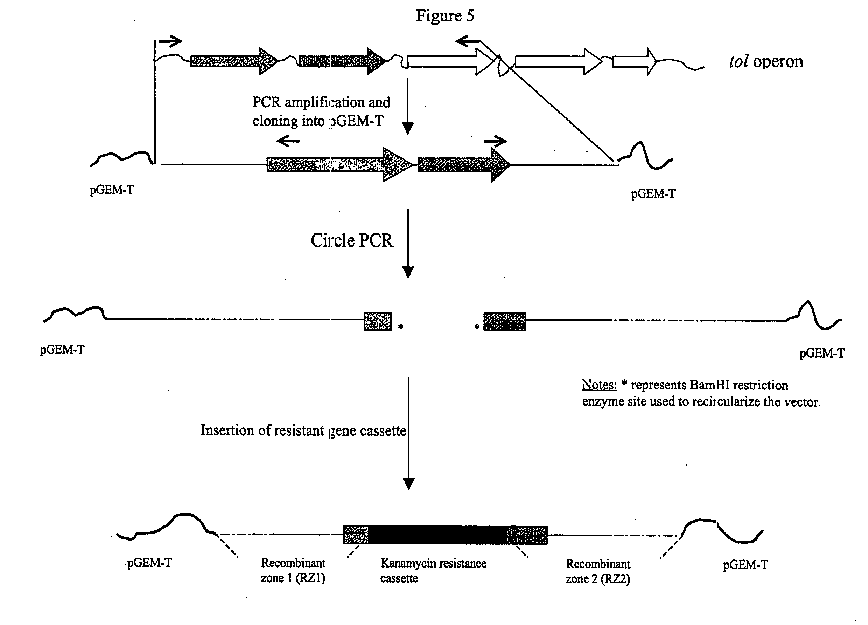

For that purpose, a mutator plasmid was constructed using *E. coli* cloning technologies. The main steps are shown in Figure 5. Briefly, genomic DNA was extracted from the *Moraxella catarrhalis* strain ATCC 43617 using the QIAGEN genomic DNA extraction kit (Qiagen Gmbh). This material was used to amplify by polymerase chain reaction (PCR) a 2151 nucleotide-DNA fragment covering 501 nucleotides upstream of the *tolQ* gene start codon (ATG) to 500 nucleotides downstream of the *tolR* stop codon (TAA) using primers A (5' - GCTCTAGAGCTTCAGCAGTCACGGGCAAATCATGATTA - 3') and B (5' - CGGAGCTCTGCTCAAGGTCTGAGACATGATTAGAATAT - 3'). This PCR product was introduced into the pGEM-T-cloning vector (Promega) according to the manufacturer's instructions. The obtained plasmid was then submitted to circle PCR mutagenesis (Jones and Winistofer, (1992), Biotechniques 12: 528-534) in order to delete the *tol QR* genes (consisting of an amplification of the entire vector without the region comprised between the two primers). The circle PCR was performed using primers C (5' - CGGGATCCCAGCGAGATTAGGCTAATGGATTCGTTCA - 3') and D (5'- CGGGATCCAATGTTGGTATCACCCAAGTGAGTTTGCTT - 3') hybridizing 31 nucleotides downstream of the start codon (ATG) of *tolQ* and 48bp upstream of the stop codon (TAA) of *tolR,* respectively (see Figure 5). Both primers contain a *Bam*HI restriction site (underlined). The obtained PCR fragment was then purified using the PCR Clean Up Kit (Boehrmgcr), digested by *Bam*HIand ligated resulting in a plasmid carrying a 532 nucleotide- 5' flanking sequence and a 548 nucleotide-3' flanking sequence separated by a *Bam*HI restriction site. Kanamycin resistance cassettes were then introduced into the *Bam*HI site in order to be able to select recombinants in the host bacteria. Two different cassettes were subcloned giving two different plasmids, one was the kanamycin resistance gene from Tn903 (KanR) subcloned from plasmid pUC4K (Amersham Pharmacia Biotech) and the other was a *sacB-neo* cassette originating from pIB279 carrying the kanamycin resistance gene from Tn*5* and the *sacB* gene (Blomfield et al., (1991), Molecular Microbiology, 5: 1447-1457). *sacB* is a counter-selection marker deleterious for bacteria in the presence of sucrose and allows further pushing-out of the cassette. Both cassettes were subcloned using the available *Bam*HI restriction sites. The sequences of the obtained clones have been confirmed using Big Dye Cycle Sequencing kit (Perkin Eimer) and an ABI 373A/PRISM DNA sequencer. Alternatively, the pKNG101 suicide vector can be used to introduce the mutation after subcloning the flanking regions into the multi-cloning site of the vector (Kaniga et al., (1991), Gene 109:137-141).

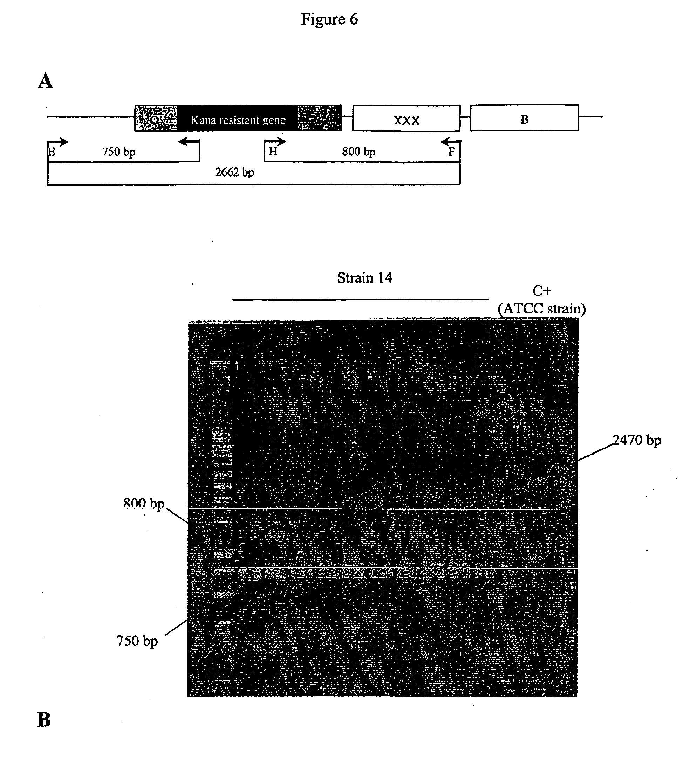

The plasmid carrying the kanamycin resistance marker from Tn*903* was used to transform *Moraxella catarrhalis* strain 14 isolated from human nasopharynx in Oslo, Norway. The transformation technique is based on the natural DNA uptake competence of the strain. - 10 bacterial colonies were mixed with 25 µg of DNA (in 20 µl PBS) and incubated for three hours at 36 °C. Recombinant *Moraxella catarrhalis* clones were then selected on Muller-Hinton plates containing 20 µg/ml kanamycin and mutants resulting from a double recombinant event were screened by PCR using primers E (5'- ATCGGCGTGGCTGTGTGTGGC - 3'), F (5'-ACCGAATTGGATTGAGGTCAC - 3'), G (5'- GCGATTCAGGCCTGGTATGAG - 3') and H (5'- TTGTGCAATGTAACATCAGAG - 3'). Following thermal amplification, a ∼10µl aliquot of the reaction was analyzed by agarose gel electrophoresis (1% agarose in a Tris-borate-EDTA (TBE) buffer). DNA fragments were visualized by UV illumination after gel electrophoresis and ethidium bromide staining. A DNA molecular size standard (Smartladder, Eurogentec) was electophoresed in parallel with the test samples and was used to estimate the size of the PCR products. As shown in Figure 6, several transformants produced the expected size PCR product and were identified as *tolQR Moraxella catarrhalis* mutant strains. Sequencing confirmed correct integration of the cassette. These clones can be tested for outer membrane vesicles production.

### Example 3. Mutation of ompCD from Moraxella catarrhalis

The aim of the experiment was to mutate the *omp*CD gene from *Moraxella catarrhalis* into a truncated gene without the peptidoglycan-associated 3' - coding region in order to obtain a hyperblebbing *Moraxella* strain. In this experiment, a stop codon was introduced after the phenylalanine at the end of the transmembrane domain of the protein.

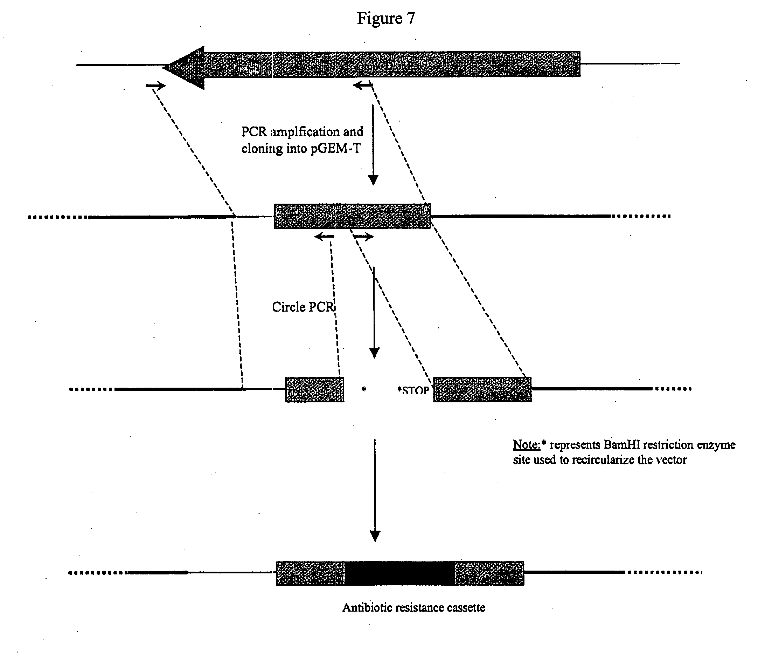

For that purpose, a mutator plasmid was constructed using *E. coli* cloning technologies. The main steps are shown in Figure 7. Briefly, genomic DNA was extracted from the *Moraxella catarrhalis* strain ATCC 43617 using the QIAGEN genomic DNA extraction kit (Qiagen Gmbh). This material was used to amplify by polymerase chain reaction (PCR) a 1000 nucleotide-DNA fragment covering 500 nucleotides upstream and downstream of the critical phenylalanine residue, using primers 1 (5' - CCTCTAGACGCTTATTATAACATAAATCAGTCTAACTG - 3') and 2 (5' - AAGGTACCAGCAGAAGTAGCCAATGGGCAAAACATTGC - 3'). This PCR product was introduced into the pGEM-T cloning vector (Promega) according to the manufacturer's instructions. The obtained plasmid was then submitted to circle PCR mutagenesis (Jones and Winistofer, (1992), Biotechniques 12: 528-534) in order to introduce a stop codon and a *Bam*HI restriction site. The circle PCR was performed using primers 3 (5' - CCGGATCC**TTA**ACGGTATTGTGGTTTGATGATTGATTT - 3') and 4 (5' - AAGGATCCGCGCAAATGCGTGAATTCCCAAATGCAACT - 3') hybridizing 62 nucleotides upstream and 39 nucleotides downstream the TTC codon encoding the phenylalanine (Figure 7). Both primers contain a *Bam*HI restriction site (underlined) and primer 3 also contains the stop codon (bold). The obtained PCR fragment was then purified using the PCR Clean Up Kit (Boehringer), digested by *Bam*HI and ligated resulting in a plasmid carrying a 438 nucleotide - 5' flanking sequence and 540 nucleotide-3' flanking sequence separated by a *BamHI* site. Kanamycin resistance cassettes were then introduced into the BamHI site in order to be able to select recombinants in the host bacteria. Two different cassettes were subcloned giving two different plasmids, one was the kanamycin resistance gene from Tn*903* (KanR) subcloned from plasmid pUC4K (Amersham Pharmacia Biotech) and the other was a SacB-neo cassette originating from pIB 179 carrying the kanamycin resistance gene from Tn*5* and the *sacB* gene (Blomfield et al., (1991), Molecular Microbiology,5: 1447-1457). *sacB* is a counter-selection marker deleterious for bacteria in the presence of sucrose and allows further pushing-out of the cassette. Both cassettes were subcloned using the available BamHI restrictions sites. The sequences of the obtained clones were confirmed using Big Dye Sequencing kit (Perkin Elmer) and an ABI 373A/PRISM DNA sequencer. Alternatively, the pKNG101 suicide vector can be used to introduce the mutation after subcloning the flanking regions into the multi-cloning site of the vector (Kaniga et al., (1991), Gene 109:137-141).

The plasmid carrying the kanamycin resistance marker from Tn*903* can be used to transform *Moraxella catarrhalis.* Recombinant *Moraxella catarrhalis* clones can be selected on Muller-Hinton plates containing 20 µg/ml kanamycin and mutants resulting from a double recombinant event can be screened by PCR. These clones can then be tested for outer membrane vesicles production.

### Example 4. Deletion of the tolQR genes in non-typeable Haemophilus influenzae

The aim of the experiment was to delete the *tolQR* genes from non-typeable *Haemophilus influenzae* (NTHI) in order to obtain a hyperblebbing strain.

For that purpose, a mutator plasmid was constructed using *E. coli* cloning technologies. The main steps are shown in Figure 5. Briefly, genomic DNA was extracted from the non-typeable *Haemophilus influenzae* strain 3224A using the QIAGEN genomic DNA extraction kit (Qiagen Gmbh). This material was used to amplify by polymerase chain reaction (PCR) a 1746 nucleotide-DNA fragment covering 206 nucleotides upstream of the *tolQ* gene codon to 364 nucleotides downstream of the *tolR* stop codon using primers ZRI-EcoRI (5' - CCGGAATTCAAAGTGCGGTAGATTTAGTCGTAGTAATTGATTTACTTATG - 3') and ZR2-XbaI (5' - CTAGTCTAGAACGTTGCTGTTCTTGCTG - 3'). This PCR product was introduced into the pGEM-T cloning vector (Promega) according to the manufacturer's instructions. The obtained plasmid was then submitted to circle PCR mutagenesis (Jones and Winistofer, (1992), Biotechniques 12: 528-534) in order to delete the *tol QR* genes (consisting of an amplification of the entire vector without the region comprised between the two primers). The circle PCR was performed using primers ZR1-BamHI (5' - CGCGGATCCCGCTTCAGGTGCATCTGG - 3') and ZR2-BamHI (5' - CGCGGATCCAGACAGGAATTTGATAAGG - 3') hybridizing 312 nucleotides downstream of the start codon of *tolQ* and 144 bp upstream of the stop codon of *tolR,* respectively (Figure 5). Both primers contain a *BamHI* restriction site (underlined). The obtained PCR fragment was then purified using the PCR Clean Up Kit (Boehringer), digested by *Bam*HI and ligated resulting in a plasmid carrying a 517 nucleotide-5' flanking sequence and a 507 nucleotide- 3' flanking region separated by a *Bam*HI restriction site. Kanamycin resistance cassettes were then introduced into the *Bam*HI site in order to be able to select recombinants in the host bacteria. Two different cassettes were subcloned giving two different plasmids, one was the kanamycin resistance gene from Tn*903* (KanR) subcloned from plasmid pUC4K (Amersham Pharmacia Biotech) and the other was a *sacB-neo* cassette originating from pIB279 carrying the kanamycin resistance gene from Tn*5* and the *sacB* gene (Blomfield et al., (1991), Molecular Microbiology,5: 1447-1457). *sacB* is a counter-selection marker deleterious for bacteria in the presence of sucrose and allows further pushing-out of the cassette. Both cassettes were subcloned using the available *Bam*HI restriction sites. The sequences of the obtained clones have been confirmed using Big Dye Cycle Sequencing kit (Perkin Elmer) and an ABI 373A/PRISM DNA sequencer. Alternatively, the pKNG101 suicide vector can be used to introduce the mutation after subcloning the flanking regions into the multi-cloning site of the vector (Kaniga et al., (1991), Gene 109:137-141).

The plasmid carrying the kanamycin resistance marker from Tn903 was used to transform non-typeable *Haemophilus influenzae* strain 3224A. Transformation was realized using competent NTHI cells obtained by a calcium chloride treatment according to Methods in Enzymology, Bacterial genetic systems, ed. J. H. Miller, Academic Press Inc., vol. 204, p. 334. Recombinant non-typeable *Haemophilus influenzae* clones were selected on GC plates containing 15 µg/ml kanamycin and mutants resulting from a double recombinant event were screened by PCR using primers NTHI-Fo-ZR1 (5' - CCTTACTAGAGGAACAACAACTC - 3'), NTHI-RE-ZR2 (5' - GCCTCTTCAGCTTGCTTCTG - 3'), ZR1-EcoRI (5' - CCGCTAATTCAAAGTGCGGTAGATTTAGTCGTAGTAATTGATTTACTTATG - 3') and ZR2-XbaI (5' - CTAGTCTAGAACGTTGCTGTTCTTGCTG - 3'). Following thermal amplification, a ∼10 µl aliquot of the reaction was analyzed by agarose gel electrophoresis (1% agarose in a Tris-borate-EDTA (TBE) buffer). DNA fragments were visualized by UV illumination after gel electrophoresis and ethidium bromide staining. A DNA molecular size standard (Smartladder, Eurogentec) was electrophoresed in parallel with the test samples and was used to estimate the size of the PCR products. Several transformants produced the expected size PCR product and were identified as non-typeable *Haemophilus influenzae* mutant strains carrying the antibiotic resistance cassette.

### Example 5. Deletion of the tolRA genes in non-typeable Haemophilus influenzae

The aim of the experiment was to delete the *tolRA* genes from *non-typeable Haemophilus influenzae* (NTHI) in order to obtain a hyperblebbing strain.

For that purpose, a mutator plasmid was constructed using *E*. *coli* cloning technologies. The main steps are shown in Figure 5. Briefly, genomic DNA was extracted from the non-typeable *Haemophilus influenzae* strain 3224A using the QIAGEN genomic DNA extraction kit (Qiagen Gmbh). This material was used to amplify by polymerase chain reaction (PCR) a 1797 nucleotide-DNA fragment covering 244 nucleotides upstream of the *tolR* gene codon to the *tolA* stop codon using primers ZR5-EcoRI (5'-CCGGAATTCAAAGTGCGGTAGATTTAGTCGTAATTCGCTGAGGCC - 3') and ZR6-XbaI (5' - CTAGTCTAGATTATCGAATATCAAAGTCAATAATG - 3'). This PCR product was introduced into the pGEM-T cloning vector (Promega) according to the manufacturer's instructions. The obtained plasmid was then submitted to circle PCR mutagenesis (Jones and Winistofer, (1992), Biotechniques 12: 528-534) in order to delete the *tolRA* genes (consisting of an amplification of the entire vector without the region comprised between the two primers). The circle PCR was performed using primers ZR5-BamHI (5' - CGCGGATCCTTCTTCTGTTTAAACCTTCTTG - 3') and ZR6-BamHI (5' - CGCGGATCCAAGCAAAGGCTGAAGCGG - 3') hybridizing 257 nucleotides downstream of the start codon of *tolR* and 500 nucleotides upstream of the stop codon of *tolA,* respectively (see Figure 5). Both primers contain a *Bam*HI restriction site (underlined). The obtained PCR fragment was then purified using the PCR Clean Up Kit (Boehringer), digested by *Bam*HI and ligated resulting in a plasmid carrying a 502 nucleotide-5' flanking sequence and a 500 nucleotide-3' flanking sequence separated by a *Bam*HI restriction site. Kanamycin resistance cassettes were then introduced into the *Bam*HI site in order to be able to select recombinants in the host bacteria. Two different cassettes were subcloned giving two different plasmids, one was the kanamycin resistance gene from Tn*903* (KanR) subcloned from plasmid pUC4K (Amersham Pharmacia Biotehc) and the other was a *sacB-neo* cassette originating from pIB279 carrying the kanamycin resistance gene from Tn*5* and the *sacB* gene (Blomfield et al., (1991), Molecular Microbiology,5: 1447-1457). *sacB* is a counter-selection marker deleterious for bacteria in the presence of sucrose and allows further pushing-out of the cassette. Both cassettes were subcloned using the available *BamHI* restriction sites. The sequences of the obtained clones have been confirmed using Big Dye Cycle Sequencing kit (Perkin Elmer) and an ABI 373A/PRISM DNA sequencer. Alternatively, the pKNG101 suicide vector can be used to introduce the mutation after subcloning the flanking regions into the multi-cloning site of the vector (Kaniga et al., (1991), Gene 109:137-141).

The plasmid carrying the kanamycin resistance marker from Tn*903* was used to transform non-typeable *Haemophilus influenzae* strain 3224. Transformation was realized using competent NTHI cells obtained by a calcium chloride treatment according to Methods in Enzymology, Bacterial genetic systems, ed. J. H. Miller, Academic Press Inc., vol. 204, p. 334. Recombinant non-typeable *Haemophilus influenzae* clones were selected on GC plates containing 15 µg/ml kanamycin and mutants resulting from a double recombinant event were screened by PCR using primers NTHI-FO-ZR5 (5' - CGCTGAGGCCTTGATTGC - 3'), NTHI-RE-ZR6 (5' - GTACAATCGCGAATACGCTCAC - 3'), ZR5-EcoRI (5' - CCGGAATTCAAAGTGCGGTAGATTTAGTCGTAATTCGCTGAGGCC - 3') and ZR6-XbaI (5' - CTAGTCTAGATTATCGAATATCAAAGTCAATAATG - 3'). Following thermal amplification, a ∼10 µl aliquot of the reaction was analyzed by agarose gel electrophoresis (1% agarose in a Tris-borate-EDTA (TBE) buffer). DNA fragments were visualized by UV illumination after gel electrophoresis and ethidium bromide staining. A DNA molecular size standard (Smartladder, Eurogentec) was electrophoresed in parallel with the test samples and was used to estimate the size of the PCR products. Several transformants produced the expected size PCR product and were identified as non-typeable *Haemophilus influenzae* mutant strains carrying the antibiotic resistance cassette.

### Example 6. Mutation of P5 gene in non-typeable Haemophilus influenzae

The aim of the experiment was to mutate the P5 gene from *Haemophilus influenzae* (NTHI) into a truncated gene without the peptidoglycan-associated 3' - coding region in order to obtain a hyperblebbing NTHI strain. In this experiment, a stop codon was introduced after the phenylalanine at the end of the transmembrane domain of the protein.

For that purpose, a mutator plasmid was constructed using *E. coli* cloning techologies. The main steps are shown Figure 7. Briefly, genomic was extracted from the non-typeable *Haemophilus influenzae* strain 3224A using the QIAGEN genomic DNA extraction kit (Qiagen Gmbh). This material was used to amplify by polymerase chain reaction (PCR) a 1047 nucleotide-DNA fragment upstream and downstream of the TTT codon encoding the critical phenylalanine residue, using primers P5-01 bis (5'-GATGAATTCAAAGTGCGGTAGATTTAGTCGTAGTAATTAATAACTTA - 3') and P5-02 (5' - CTAGTCTAGAAGGTTTCCATAATGTTTCCTA - 3'). This PCR product was introduced into the pGEM-T cloning vector (Promega) according to the manufacturer's instructions. The obtained plasmid was then submitted to circle PCR mutagenesis (Jones and Winistofer, (1992), Biotechniques 12: 528-534) in order to introduce a stop codon and a *BamHI* restriction site. The circle PCR was performed using primers P5-03 (5' - CGCGGATCCCTAAAAAGTTACATCAGAATTTAAGC - 3') and P5-04 (5' - CGCGGATCCGCATTTGGTAAAGCAAACTT - 3') hybridizing exactly at the TTT codon encoding the phenylalanine (see Figure 7). Both primers contain a *BamHI* restriction site (underlined) and primer 3 also contains the stop codon (bold). The obtained PCR fragment was then purified using the PCR Clean Up Kit (Boehring), digested by *Bam*HI and ligated resulting in a plasmid carrying a 518 nucleotide-5' flanking sequence and a 538 nucleotide -3' flanking sequence separated by a *Bam*HI restriction site. Kanamycin resistance cassettes were then introduced into the *Bam*HI site in order to be able to select recombinants in the host bacteria. Two different cassettes were subcloned giving two different plasmids, one was the kanamycin resistance gene from Tn*903* (KanR) subcloned from plasmid pUC4K (Amersham Pharmacia Biotech) and the other was a *sacB-neo* cassette originating from pIB279 carrying the kanamycin resistance gene from Tn*5* and the *sacB* gene (Blomfield et al., (1991), Molecular Microbiology,5: 1447-1457). *sacB* is a counter-selection marker deleterious for bacteria in the presence of sucrose and allows further pushing-out of the cassette. Both cassettes were subcloned using the available *Bam*HI restriction sites. The sequences of the obtained clones were confirmed using Big Dye Cycle Sequencing kit (Perkin Elmer) and an ABI 373A/PRISM DNA sequencer. Alternatively, the pKNG101 suicide vector can be used to introduce the mutation after subcloning the flanking regions into the multi-cloning site of the vector (Kaniga et al., (1991), Gene 109:137-141).

The plasmid carrying the kanamycin resistance marker from Tn*903* was used to transform non-typeable *Haemophilus influenzae* strain 3224. Transformation was realized using competent NTHI cells obtained by a calcium chloride treatment according to Methods in Enzymology, Bacterial genetic systems, ed. J. H. Miller, Academic Press Inc., vol. 204, p. 334. Recombinant non-typeable *Haemophilus influenzae* clones were selected on GC plates containing 15 µg/ml kanamycin and mutants resulting from a double recombinant event were screened by PCR using primers P5-01 bis (5'-GATGAATTCAAAGTGCGGTAGATTTAGTCGTAGTAATTAATAACTTA - 3') and P5-02 (5' - CTAGTCTAGAAGGTTTCCATAATGTTTCCTA - 3'). Following thermal amplification, a ∼10 µl aliquot of the reaction was analyzed by agarose gel electrophoresis (1% agarose in a Tris-borate-EDTA (TBE) buffer). DNA fragments were visualized by UV illumination after gel electrophoresis and ethidium bromide staining. A DNA molecular size standard (Smartladder, Eurogentec) was electrophoresed in parallel with the test samples and was used to estimate the size of the PCR products. Several transformants produced the expected size PCR product and were identified as non-typeable *Haemophilus influenzae* mutant strains carrying the antibiotic resistance cassette.

SEQ. ID NO:1
Nucleotide sequence of the coding region of exbB from Neisseria meningitidis (serogroup B) – strain MC58

Accession N° and sequences (DNA & protein) of NmB strain ExbB

>NMB1729
ATGAATTTGAAATTAGTGTTTGAATCGGGCGATCCCGTCCTGATTGGTGTGTTTGTGTTG
ATGCTGTTGATGAGTATCGTAACGTGGTGTTTGGTTGTCTTGCGCTGCATCAAGCTGTAT
CGGGCGCGCAAAGGGAATGCCGCCGTCAAACGGCATATGCGCGATACTTTGTCGCTGAAC
GACGCGGTCGAAAAAGTGCGCGCCGTCGATGCGCCTTTGTCCAAACTGGCGCAAGAGGCA
TTGCAGTCTTACCGCAACTACCGCCGAAACGAAGCGTCCGAACTGGCGCAGGCTTTGCCG
TTGAACGAGTATTTGGTCATTCAAATCCGCAACAGTATGGCGCAGATTATGCGCCGGTTT
GATTACGGGATGACCGCGCTTGCCTCCATCGGCGCGACCGCGCCGTTTATCGGGCTGTTC
GGCACGGTTTGGGGGATTTACCACGCCCTGATCAATATCGGGCAAAGCGGGCAGATGAGT
ATTGCGGCGGTTGCCGGCCCGATTGGCGAGGCACTGGTGGCGACGGCGGCGGGTTTGTTC
CTGGCCGATTCCCGCCGGTGTTGGCATACAACTTCCTCAATCGCGGCACAAAAATACTGACC
CAGGATTTGGATGCGATGGCGCACGATTTGCACGTCCGCCTGCTTAATCAAAAGGATAGC

SEQ. ID NO:2
Amino acid sequence of ExbB from Neisseria meningitidis (serogroup B) – strain MC58

>NMB1729
MNLKLVFESGDPVLIGVFVLMLLMSIVTWCLVVLRCIKLYRARKGNAAVKRHMRDTLSLN
DAVEKVRAVDAPLSKLAQEALQSYRNYRRNEASELAQALPLNEYLVIQIRNSMAQIMRRF
DYGMTALASIGATAPFIGLFGTVWGIYHALINIGQSGQMSIAAVAGPIGEALVATAAGLF
VAIPAVLAYNFLNRGTKILTQDLDAMAHDLHVRLLNQKDS

SEQ. ID NO:3
Nucleotide sequence of the coding region of exbD from Neisseria meningitidis (serogroup B) – strain MC58

Accession N° and sequences (DNA & protein) of NmB strain ExbD
>NMB1728
ATGGCATTTGGTTCGATGAATTCCGGCGACGATTCTCCGATGTCCGACATCAACGTTACG
CCGTTGGTGGACGTGATGCTGGTGTTGCTGATTGTGTTTATGATTACTATGCCGGTGCTG
ACGCATTCCATCCCTTTGGAACTGCCGACCGCGTCCGAGCAGACAAACAAGCAGGACAAA
CAGCCTAAAGACCCCCTGCGCCTGACGATTGATGCGAACGGCGGCTATTATGTCGGCGGG
GATTCTGCAAGCAAAGTGGAAATCGGGGAAGTGGAAAGCCGTCTGAAAGCCGCCAAGGAG
CAGAATGAAAACGTGATTGTGGCGATTGCGGCAGACAAGGCGGTGGAATACGATTATGTA
AACAAAGCTTTAGAAGCCGCCCGTCAGGCAGGAATCACCAAAATCGGTTTTGTAACCGAA
ACCAAGGCGCAA

SEQ. ID NO:4
Amino acid sequence of ExbD from Neisseria meningitidis (serogroup B) – strain MC58

>NMB1728
MAFGSMNSGDDSPMSDINVTPLVDVMLVLLIVFMITMPVLTHSIPLELPTASEQTNKQDK
QPKDPLRLTIDANGGYYVGGDSASKVEIGEVESRLKAAKEQNENVIVAIAADKAVEYDYV
NKALEAARQAGITKIGFVTETKAQ

SEQ. ID NO:5
Nucleotide sequence of DNA region (1000 bp ) up-stream from the exbB gene from Neisseria meningitidis (serogroup B) – strain MC58

DNA sequence of 1kb upstream of ExbB strain NmB MC58

5′-CATAATGATTCCAACACTGAAAAAACCAATCAAACATCCAAGCTGCCGCAAACCGCTGCG
ExbB <- GTA 5′

GCAACCGCCTAATTCAATTCAAACTTGACGGGGACTTTAAACTCCGTCCAGGCATTGGCT
TGAAAATGCCCGTTTTGCGCCGCCTTGCGTGCCGCATTGTCCAACCGGGAAAAACCACTG
CTTTTCACGATTTTAACGGACTCAACATGACCGCCCGGAGAAACCAAAACGCTCAAAACA
ACCGTACCCTGCTCGTCATTCTCCATAGAAAGCGTGGGATAAGCCGGGCGCGGAATGCTG

CCGTTGGCGCGTAAAGGATTGCCTTTGCTGCTGCCGGCTCCTTCCCCGTGTTCGCCTTTG
ACACCGCCGCTACCTTTACCGCTGCCTTCTCCGCGCCCCGTTCCGTCTCCTTTGGTACCA
GTTCCCTTATCTTCCCCATTGCCCTGCTCGCTGTCTGCTTTGGCAGAAGCATTGCCGGGA
TGTTCGGCAGGTTTTTCAGACGGCTTCTCGACCGGTTTTTCCGCCGGTTTCGGGACAGGC
TTCGCTTCCGGCTTAGGCTCTGGTTTCGGTTTTTCTTCGGGTTTCGGCTTTTCTTCAGGT
TTCGGCTCTTCCTTAGGCTGCTGAATATCCGCATCCGCCTTTTTCGTAACCACCGGCTTC
AAAACCGGCTTGGGCGGCTCGACAGGTTTGGGCGGCTCGGGCACGGGTTGCGGTTCGGGC
GCAGCAGGCGCGCCTGCACCTTCGGGGGCGCCGTCCCCTCCGCCAAAATCGCCCAAATCG
ACAAATTCAATAACATTGCCTGACTCTATCACGGGCAGCTTGTGCGCCTGCCAGAGCAAT
GCCACCATTGCCAAATGCAGCAGTGCGACGGAAAACACGACTGCGGGGGTTAAAATTCGT
TCTTTATCCATAATTCGGGCATAATAATAGCAACAATTCCTATTTGCAACCTATTTTTAC
AATTTTTGGTCATATGAATGTCTGTTCCGTTCACAGGCAAA-3'

SEQ. ID NO:6
Nucleotide sequence of DNA region (1000 bp ) up-stream from the exbD gene from Neisseria meningitidis (serogroup B) – strain MC58

DNA sequence of 1kb upstream of ExbD strain NmB MC58

5'-CATAATCAGCTATCCTTTTGATTAAGCAGGCGGACGTGCAAATCGTGCGCCATCGCATC
ExbD <_GTA -5'
CAAATCCTGGGTCAGTATTTTTGTGCCGCGATTGAGGAAGTTGTATGCCAACACCGCCGG
AATCGCCACGAACAAACCCGCCGCCGTCGCCACCAGTGCCTCGCCAATCGGCCGGCAAC
CGCCGCAATACTCATCTGCCCGCTTTGCCCGATATTGATCAGGGCGTGGTAAATCCCCCA
AACCGTGCCGAACAGCCCGATAAACGGCGCGGTCGCGCCGATGGAGGCAAGCGCGGTCAT
CCCGTAATCAAACCGGCGCATAATCTGCGCCATACTGTTGCGGATTTGAATGACCAAATA
CTCGTTCAACGGCAAAGCCTGCGCCAGTTCGGACGCTTCGTTTCGGCGGTAGTTGCGGTA
AGACTGCAATGCCTCTTGCGCCAGTTTGGACAAAGGCGCATCGACGGCGCGCACTTTTTC
GACCGCGTCGTTCAGCGACAAAGTATCGCGCATATGCCGTTTGACGGCGGCATTCCCTTT
GCGCGCCCGATACAGCTTGATGCAGCGCAAGACAACCAAACACCACGTTACGATACTCAT
CAACAGCATCAACACAAACACACCAATCAGGACGGGATCGCCCGATTCAAACACTAATTT
CAAATTCATAATGATTCCAACACTGAAAAAACCAATCAAACATCCAAGCTGCCGCAAACC
GCTGCGGCAACCGCCTAATTCAATTCAAACTTGACGGGGACTTTAAACTCCGTCCAGGCA
TTGGCTTGAAAATGCCCGTTTTGCGCCGCCTTGCGTGCCGCATTGTCCAACCGGGAAAAA
CCACTGCTTTTCACGATTTTAACGGACTCAACATGACCGCCCGGAGAAACCAAAACGCTC
AAAACAACCGTACCCTGCTCGTCATTCTCCATAGAAAGCGTGGGATAAGCCGGGCGCGGA
ATGCTGCCGTTGGCGCGTAAAGGATTGCCTTTGCTGCTGCCGGC-3'

SEQ. ID NO:7
Nucleotide sequence of the coding region of rmpM from Neisseria meningitidis (serogroup B) – strain MC58

Accession N° of RmpM (also called OMP4 in N. menigitidis)

Nm strain MC58 (serogroup B): (DNA & protein sequences)

>NMB0382
ATGACCAAACAGCTGAAATTAAGCGCATTATTCGTTGCATTGCTCGCTTCCGGCACTGCT
GTTGCGGGCGAGGCGTCCGTTCAGGGTTACACCGTAAGCGGCCAGTCGAACGAAATCGTA
CGCAACAACTATGGCGAATGCTGGAAAAACGCCTACTTTGATAAAGCAAGCCAAGGTCGC
GTAGAATGCGGCGATGCGGTTGCTGCCCCCGAACCCGAGCCAGAACCCGAACCCGCACCC
GCGCCTGTCGTCGTTGTGGAGCAGGCTCCGCAATATGTTGATGAAACCATTTCCCTGTCT
GCCAAAACCCTGTTCGGTTTCGATAAGGATTCATTGCGCGCCGAAGCTCAAGACAACCTG
AAAGTATTGGCGCAACGCCTGAGTCGAACCAATGTCCAATCTGTCCGCGTCGAAGGCCAT
ACCGACTTTATGGGTTCTGACAAATACAATCAGGCCCTGTCCGAACGCCGCGCATACGTA
GTGGCAAACAACCTGGTCAGCAACGGCGTACCTGTTTCTAGAATTTCTGCTGTCGGCTTG
GGCGAATCTCAAGCGCAAATGACTCAAGTTTGTGAAGCCGAAGTTGCCAAACTGGGTGCG
AAAGTCTCTAAAGCCAAAAAACGTGAGGCTCTGATTGCATGTATCGAACCTGACCGCCGT
GTGGATGTGAAAATCCGCAGCATCGTAACCCGTCAGGTTGTGCCGGCACACAATCATCAC
CAACACTAA

SEQ. ID NO:8
Amino acid sequence of RmpM from Neisseria meningitidis (serogroup B) – strain MC58

>NMB0382
MTKQLKLSALFVALLASGTAVAGEASVQGYTVSGQSNEIVRNNYGECWKNAYFDKASQGR
VECGDAVAAPEPEPEPEPAPAPVVVVEQAPQYVDETISLSAKTLFGFDKDSLRAEAQDNL
KVLAQRLSRTNVQSVRVEGHTDFMGSDKYNQALSERRAYVVANNLVSNGVPVSRISAVGL
GESQAQMTQVCEAEVAKLGAKVSKAKKREALIACIEPDRRVDVKIRSIVTRQVVPAHNHH

QH

SEQID N°9

Nucleotide sequence of DNA region (729 bp) corresponding to the rmpM gene in

the Neisseria meningitidis serogroup A strain Z2491

ATGACCAAACAGCTGAAATTAAGCGCATTATTCGTTGCATTGCTCGCTTCCGGCACTGCTGTTGCGGGCGAGGCGTCCGT
TCAGGGTTACACCGTAAGCGGCCAGTCGAACGAAATTGTACGCAACAACTATGGCGAATGCTGGAAAAACGCCTACTTTG
ATAAAGCAAGCCAAGGTCGCGTAGAATGCGGCGATGCGGTTGCTGCCCCCGAACCCGAGCCAGAACCCGAACCCGCACCC
GCGCCTGTCGTCGTTGTGGAGCAGGCTCCGCAATATGTTGATGAAACCATTTCCCTGTCTGCCAAAACCCTGTTCGGTTT
CGATAAGGATTCATTGCGCGCCGAAGCTCAAGACAACCTGAAAGTATTGGCGCAACGCCTGGGTCAAACCAATATCCAAT
CTGTCCGCGTCGAAGGCCATACCGACTTTATGGGTTCTGACAAATACAATCAGGCCCTGTCCGAACGCCGCGCATACGTA
GTGGCAAACAACCTGGTCAGCAACGGCGTACCTGTTTCTAGAATTTCTGCTGTCGGCTTGGGCGAATCTCAAGCGCAAAT
GACTCAAGTTTGTGAAGCCGAAGTTGCCAAACTGGGTGCGAAAGTCTCTAAAGCCAAAAAACGTGAGGCTCTGATTGCAT
GTATCGAACCTGACCGCCGCGTGGATGTGAAAATCCGCAGCATCGTAACCCGTCAGGTTGTGCCGGCACACAATCATCAC
CAACACTAA

SEQ. ID NO:10
Nucleotide sequence of DNA region (1000 bp ) up-stream from the rmpM gene
from Neisseria meningitidis (serogroup B) – strain MC58

DNA sequence of 1kb upstream of RmpM strain NmB MC58
AAAATGCCCGCGCGATGCTGCTGCCCGCATTGAATGCAAATTCATAAGTAATCAGCGGAA
ACCTCGCCAAATCTTCAATACGGAGGGGGTTTCTGCATTCGAGCAAGGGGTGGTCGTTCG
GTACGATAACCGCATGAGTCCAGTCATAGCAGGGAAGTTTTCCCAGTTCGGGATGGTCGT
CTATCCGTTCCGTAACAATCGCCAAGTCCGCCTCGCCTGAGGTAACCATACGTGCGATGG
CGGCAGGGCTCCCCTGTTTGATGGTCAGGTTGACTTTCGGATAGCGTTTCACAAAATCGG
CAACAATCAAGGGTAGGGCATAGCGTGCCTGAGTATGCGTCGTGGCAACCGTCAGCGAAC
CGCTGTCCTGTCCGGTAAACTCGCTGCCGATATTTTTAATGTTCTGAACATCGCGCAAAA
TACGTTCCGCAATATCCAAAACCACCTTGCCCGGCTGCGAGACCGAAACCACGCGCTTGC
CGCTGCGGATAAAAATCTGAATGCCGATTTCTTCTTCCAGCAATTTGATTTGTTTGGAGA
TGCCGGGTTGCGAAGTAAACAAGGCTTCGGCCGCTTCGGAAACGTTCAGGTTGTGCTGGT
AAACTTCTAAGGCGTATTTCAATTGTTGTAATTTCATGGCGGGTCGGTGTGGGTCTGTGT
CGGGTGGCTGAACATTGTTTATAATTTATCATATTTCTTGCCGGTACGGTATGGGGCTT
TGCCGTTGTGTTTGTTGTTTTTGTGCAACGGCAATCGTGCGATATGGAAAAAATCCCCCT
AAAGTAATGACACGGAATTGATTTTTCGGCATGATAGACTATCAGGAAACAGGCTGTTTT
ACGGTTGTTTTCAGGCGTTGAGTATTGACAGTCCGCCCCCTGCTTCTTTATAGTGGAGAC
TGAAATATCCGATTTGCCGCCATGTTTCTACAGCGGCCTGTATGTTGGCAATTCAGCAGT
TGCTTCTGTATCTGCTGTACAAATTTAATGAGGGAATAAAATGA ATG-3'
RmpM

Nucleotide sequence :

Tol Q: complement(5168..5854) below SEQ ID NO:11 - H. influenzae strain HiRD
Tol R: complement(4677..5096) below SEQ ID NO:13 - H. influenzae strain HiRD
TolA: complement(3543..4661) below SEQ ID NO:15 - H. influenzae strain HiRD
Tol B: complement(2218..3501) below SEQ ID NO:17 - H. influenzae strain HiRD

gagtttttta
2221 tttagttaag tatggagacc aagctggaaa tttaacttga ccatcacttc ctggaaggct
2281 cgccttaaag cgaccatctg cggaaaccaa ttgtagcacc tttcctaagc cctgtgtaga
2341 actataaata atcataattc catttggaga gaggcttggg ctttcgccta gaaaagatgt
2401 actaagtacc tctgaaacgc ccgttgtgag atcttgttta actacattat tgttaccatt
2461 aatcatcaca agtgtttttc catctgcact aatttgtgcg ctaccgcgac cacccactgc
2521 tgttgcacta ccaccgcttg catccattcg ataaacttgt ggcgaaccac ttctatcgga
2581 tgtaaataaa attgaatttc cgtctggcga ccacgctggt tcagtattat tacccgcacc
2641 actcgtcaat tgagtaggtg taccgccatt tgctcccata acgtaaatat tcagaacacc
2701 atcacgagaa gaagcaaaag ctaaacgaga accatctggc gaaaaggctg gtgcgccatt

2761 atgcccttga aaagatgcca ctactttacg tgcgccagaa tttaaatcct gtacaacaag
2821 ttgtgatttt ttattttcaa acgatacata agccaaacgc tggccgtctg gagaccaagc
2881 tggagacata attggttggg cactacgatt gacgataaat tgattatagc catcataatc
2941 tgctacacga acttcataag gttgcgaacc gccatttttt tgcacaacat aagcgatacg
3001 agttctaaag gcaccacgga tcgcagttaa tttttcaaaa acttcatcgc tcacagtatg
3061 cgcgccatag cgtaaccatt tatttgttac tgtatagcta ttttgcatta atacagtccc
3121 tggcgtacct gatgcaccaa ccgtatcaat taattgataa gtaatactat aaccattacc
3181 cgatggaacc acttgcccaa ttacaattgc gtcaattcca atattcgacc aagcctcagg
3241 atttacctct gcagctgaag ttgggcgttg aggcatttga gaaaccgcaa taggattaaa
3301 cttaccactg ttacgtaaat catctgcaac aattttacta atatcttctg gtgcagaacc
3361 aacaaatggc acgacagcaa taggacgcgc accatcaacc ccttcatcaa tgacaatgcg
3421 tacttcatcg ccagcgaatg cattgcttcc aacagcaagt acaatcgcga atacgctcac
3481 taaacgtttt aataatttca ttttgttacc tttaaaattt aacaataaat tttctaaag
3541 aattatcgaa tatcaaagtc aataattggt gatttatatt tttcataaat ttcatctgat
3601 ggcgcagctg gaactttttt cgttctagcc accgcactta atgcagctga acaaatatca
3661 tcagagcctg aaattttttg ataccccaag attgtgccat ctcgacctaa ttgaatttta
3721 atacgacaaa cctttcctgc aaaatttgga tcttttaaga aacgacgttg aatctctttc
3781 ttaattacac ctgcgtattg atccccaacc ttaccaccat cgccagagcc aagtgcagca
3841 ccgctacctt gagttccacc tttatttgtg tttccccctt tagatgcact accgccacca
3901 atatctccgc catttaagaa atcatctagg cttgcttgat ctgctttacg tttcgcttcc
3961 gtagcagctt tagcttctgc atcagctttt gcttttgcct ctgctgccgc tttcgctttt
4021 gcctccgctt cagcctttgc tttagcttca gcaacggctt ttgccttagc ttcggcttct
4081 agtttcgcct tagcttccgc ctcttgtttt gctttttgag cagcaatttc tgctgctttc
4141 gctttagcct cttcttcagc ttgttttgcc gcggcagcta aacgtttagc ctctgcatct
4201 gcttttaatt ttgcagcttc agccgcttgt ttagccttag cctcttcagc ttgcttctgt
4261 ttttccaacg cttcttgacg agcttgctct tgttgttttt ttatttcttg ctgacgttgc
4321 tgttcttgct gacgttttaa ctcttcttgt cgttgaactt cttgttgatg cttaatctct
4381 tcttgattag gctcaggtgg tttttcttcc acaacaggtt ctgggcgttt ttgtttatcc
4441 gcttgccctt ttttttgttg ttgaatacgc ccccattcct gagcagccgt accagtatca
4501 acaatcactg cccctattac atctccttca ccttctccac cacccataat ttcaacagtg
4561 tgataaagtg agcttaaaat caataagcca aacaagataa agtgcaaaag gatagaaata
4621 gcaaaagcat tgattccttt cttttgtcga ttattttgca cgtgttacct acttagctaa
4681 atgggatttg tcattaaacc tacagattta atgcctgcaa gatgaagtaa attcaatgcc
4741 ttaatcactt cttcataagg tacttcttta gctccgccta ctaaaaatag cgtattatta
4801 tccttatcaa attcctgtct agataattga gtaaccattt cttctgttaa accttcttga
4861 cgttctccgc caatagaaat cgcatatttt ccaatgcctg ccacttcaag aatgacgggt
4921 actttatctt cattagaaac ctcttggctt tgcacagaat caggcaattc aacttgaacg
4981 ctttgactaa taataggggc ggttgccata aaaattaaca ctaaaactaa aagcacatct
5041 aaaaaaggca caatattaat ttcagattta attgctttac gctgacgacg agccatatat
5101 tcctctaaaa ttttaactta tttttaccgc actttttctt caaagtgcgg tcaattttcc
5161 ctatatttta gtgaggggct ttaccaaagg cttgacggtg taaaatcgtc gtaaattcat
5221 caataaaatt accgtaatct tgttcaatgg cattcactcg taagcttaaa cggttataag
5281 ccattactgc aggaattgcg gcaaataaac caatcgcagt ggcaatcaag gcctcagcga
5341 tacctggcgc taccatctgt aacgttgctt gttttgcacc acttaatgcc ataaaagcgt
5401 gcatgatacc ccaaacagtg ccgaataaac caatataagg gctaacagat gccactgtgg
5461 ctaaaaatgg aactcggttt tccaaacttt caatctcacg gttcatcgca agattcatcg
5521 cgcgcattgt gcctttaata atcgcttcag gtgcatctgg atttacttgt tttaaacgtg
5581 aaaattcttt aaatcccacg caaaaaattt gttcgctgcc cgttaatcca tcgcgacgat
5641 tagatagccc ttcataaagt ttatttaaat cttctcctga ccagaaacga tcttcaaacg
5701 tacgcgcttc ttttaaggca ttcgttaaaa tacgactacg ttgaatgata attgcccaag
5761 atatgattga gaaagaaatc aaaatcacaa ttaccagttg cacaacaata cttgctttta
5821 gaaaaagatc taaaaaattc aattctgcag tcattgcata

SEQ ID NO: 12 - TolQ amino acid sequence - H. influenzae strain HiRD

MTAELNFLDLFLKASIVVQLVIVILISFSIISWAIIIQRSRILT
NALKEARTFEDRFWSGEDLNKLYEGLSNRRDGLTGSEQIFCVGFKEFSRLKQVNPDAP
EAIIKGTMRAMNLAMNREIESLENRVPFLATVASVSPYIGLFGTVWGIMHAFMALSGA
KQATLQMVAPGIAEALIATAIGLFAAIPAVMAYNRLSLRVNAIEQDYGNFIDEFTTIL
HRQAFGKAPH

SEQ ID NO: 14 - TolR amino acid sequence - H. influenzae strain HiRd

MARRQRKAIKSEINIVPFLDVLLVLVLIFMATAPIISQSVQVEL
PDSVQSQEVSNEDKVPVILEVAGIGKYAISIGGERQEGLTEEMVTQLSRQEFDKDNNT
LFLVGGAKEVPYEEVIKALNLLHLAGIKSVGLMTNPI

SEQ ID NO: 16 - TolA amino acid sequence - H. influenzae strain HiRD

MQNNRQKKGINAFAISILLHFILFGLLILSSLYHTVEIMGGGEG
EGDVIGAVIVDTGTAAQEWGRIQQQKKGQADKQKRPEPVVEEKPPEPNQEEIKHQQEV
QRQEELKRQQEQQRQQEIKKQQEQARQEALEKQKQAEEAKAKQAAEAAKLKADAEAKR
LAAAAKQAEEEAKAKAAEIAAQKAKQEAEAKAKLEAEAKAKAVAEAKAKAEAEAKAKA
AAEAKAKADAEAKAATEAKRKADQASLDDFLNGGDIGGGSASKGGNTNKGGTQGSGAA
LGSGDGGKVGDQYAGVIKKEIQRRFLKDPNFAGKVCRIKIQLGRDGTILGYQKISGSD
DICSAALSAVARTKKVPAAPSDEIYEKYKSPIIDFDIR

SEQ ID NO: 18 - TolB amino acid sequence - H. influenzae strain HiRD

MKLLKRLVSVFAIVLAVGSNAFAGDEVRIVIDEGVDGARPIAVV
PFVGSAPEDISKIVADDLRNSGKFNPIAVSQMPQRPTSAAEVNPEAWSNIGIDAIVIG
QVVPSGNGYSITYQLIDTVGASGTPGTVLMQNSYTVTNKWLRYGAHTVSDEVFEKLTA
IRGAFRTRIAYVVQKNGGSQPYEVRVADYDGYNQFIVNRSAQPIMSPAWSPDGQRLAY
VSFENKKSQLVVQDLNSGARKVVASFQGHNGAPAFSPDGSRLAFASSRDGVLNIYVMG
ANGGTPTQLTSGAGNNTEPAWSPDGNSILFTSDRSGSPQVYRMDASGGSATAVGGRGS
AQISADGKTLVMINGNNNVVKQDLTTGVSEVLSTSFLGESPSLSPNGIMIIYSSTQGL
GKVLQLVSADGRFKASLPGSDGQVKFPAWSPYLTK

SEQ. ID NO:19
Nucleotide sequence of DNA region (1000 bp ) up-stream from the TolQRAB operon from H. influenzae – strain HiRD.

Upstream promoter sequence (1000 nt): complementary seq (atg in bold)

tcattgcata ctccgaaaaa ttattttaag 5881
tgatgaaacg ccgctttaac ttctttggga aacgccactg gtttcatctt gcctagatca 5941
acacaggcta ccttaacagt agcctttgat aacatcaggg tgttgcgcat cagtctctgt 6001
tcaaaaagga ttgtagcccc ttttacttct gaaacctctg tttccaccat aagtaaatca 6061
tccaattttg ctgccacgca ataatcaatg gcgagcgttt tgacaacaaa tgcgagttgt 6121
tgttcctcta gtaaggtttg ttgcgtaaaa tttaatgtac gcaaatattc tgttcttgct 6181
cgttcaaaaa aatgcaaata gcgagcgtga tacactacgc cacctgcatc agtatcttca 6241
taatacacac gaacaggaaa agaaaagcca ttatccaaca tattctcacc caattggtcg 6301
caataaaccg tgtattctag aaccagtttt tgggataagc aagctatcta tgaaaaactc 6361
aataagattt tattcatttt aaaacatcta aaatttttac cgcactttta gcctgactag 6421
caaaagataa ggtaatgaca aatcattttt aacctttctc attgagtaaa atctattcaa 6481
aacataaccg ttctttaaaa atagcctcta tgtaatctta agccaccagt atttttattc 6541
ttgatattta gcgtttctat gcgacaatct ttgcggttat ttactttaaa aatatgtttt 6601
actagatgga ttacgaaaat caaattgcca atattttctc actaaatggc gaattaagcc 6661

aaaatatcaa aggttttcgt cctcgagctg aacaacttga aatggcatat gctgtaggta 6721
aagcaattca aaataaatct tcccttgtta ttgaagctgg aacgggtaca ggaaaaacct 6781
ttgcatatct cgcacctgct ttagtttttg gtaaaaaaac

SEQ. ID NO:20
Nucleotide sequence of the coding region of P5 from non-typeable H. influenzae.

ATGAAAAAAACTGCAATCGCATTAGTAGTTGCTGGTTTAGCAGCAGCTTCAGT
AGCTCAAGCAGCTCCACAAGAAAACACTTTCTACGCTGGCGTTAAAGCTGGTC
AAGCATCTTTTCACGATGGACTTCGTGCTCTAGCTCGTGAAAAGAATGTTGGT
TATCACCGTAATTCTTTCACTTATGGTGTATTCGGTGGTTATCAAATTTTAAA
TCAAAATAACTTAGGTTTAGCGGTTGAATTAGGTTACGACGATTTCGGTCGTG
CCAAAGGTCGTGAAAAAGGTAGAACTGTTGCTAAACACACTAACCACGGTGCG
CATTTAAGCTTANAAGGTAGCTATGAAGTGTTAGAAGGTTTAGATGTTTATGG
TAAAGCAGGTGTTGCTTTAGTTCGTTCTGACTATAAATTGTACAATAAAAATA
GTAGTACTCTTAAAGACCTAGGCGAACATCACAGAGCACGTGCCTCTGGTTTA
TTTGCAGTAGGTGCAGAATATGCAGTATTACCAGAATTAGCAGTTCGTTTAGA
ATACCAATGGCTAACTCGCGTAGGTAAATACCGCCCTCAAGATAAACCAAATA
CCGCAATTAACTACAACCCTTGGATTGGTTCTATCAACGCAGGTATTTCTTAC
CGCTTTGGTCAAGGCGAAGCACCAGTTGTTGCAGCACCTGAAATGGTAAGCAA
AACTTTCAGCTTAAATTCTGATGTAACTTTTGCATTTGGTAAAGCAAACTTAA
AACCTCAAGCGCAAGCAACATTAGACAGCGTCTATGGCGAAATTTCACAAGTT
AAAAGTGCAAAAGTAGCGGTTGCTGGTTACACTGACCGTATTGGTTCTGACGC
GTTCAACGTAAAACTTTCTCAAGAACGTGCAGATTCAGTAGCTAACTACTTTG
TTGCTAAAGGTGTTGCTGCAGACGCAATCTCTGCAACTGGTTACGGTGAAGCA
AACCCAGTAACTGGCGCAACTTGTGACCAAGTTAAAGGTCGTAAAGCACTTAT
CGCTTGTCTTGCTCCAGACCGTCGTGTAGAAATCGCGGTAAACGGTACTAAA

SEQ. ID NO:21
Amino acid sequence of P5 from non-typeable H. influenzae.

MKKTAIALVVAGLAAASVAQAAPQENTFYAGVKAGQASFHDGLRALAREKNVG
YHRNSFTYGVFGGYQILNQNNLGLAVELGYDDFGRAKGREKGRTVAKHTNHGA
HLSLXGSYEVLEGLDVYGKAGVALVRSDYKLYNKNSSTLKDLGEHHRARASGL
FAVGAEYAVLPELAVRLEYQWLTRVGKYRPQDKPNTAINYNPWIGSINAGISY
RFGQGEAPVVAAPEMVSKTFSLNSDVTFAFGKANLKPQAQATLDSVYGEISQV
KSAKVAVAGYTDRIGSDAFNVKLSQERADSVANYFVAKGVAADAISATGYGEA
NPVTGATCDQVKGRKALIACLAPDRRVEIAVNGTK

SEQ. ID NO:22
Nucleotide sequence of the coding region of P6 from H. influenzae strain HiRD.

atgaacaaatttgttaaatcattattagttgcaggttctgtagctgcattagcagcttgtagttcatct
aacaacgatgc
tgcaggcaatggtgctgctcaaacttttggcggttactctgttgctgatcttcaacaacgttacaatac
cgtttatttcg
gttttgataaatatgacattactggtgaatacgttcaaatcttagacgcgcacgctgcatatttaaatg
caacgccagct
gctaaagtattagtagaaggtaacactgatgaacgtggtacaccagaatacaacatcgcattaggccaa
cgtcgtgcaga
tgcagttaaaggttatttagctggtaaaggtgttgatgctggtaaattaggcacagtatcttacggtga
agaaaaacctg
cagtattaggtcatgatgaagctgcatattctaaaaaccgtcgtgcagtgttagcgtac

SEQ. ID NO:23
Amino acid sequence of P6 from H. influenzae strain HiRD.

MNKFVKSLLVAGSVAALAACSSSNNDAAGNGAAQTFGGYSVADLQQRYNTVYF

GFDKYDITGEYVQILDAHAAYLNATPAAKVLVEGNTDERGTPEYNIALGQRRA
DAVKGYLAGKGVDAGKLGTVSYGEEKPAVLGHDEAAYSKNRRAVLAY

SEQ. ID NO:24
Nucleotide sequence of the coding region of P6 from non-typeable H. influenzae.

>p6nthipatent.SEQ
ATGAACAAATTTGTTAAATCATTATTAGTTGCAGGTTCTGTAGCTGCATTAGCG
GCTTGTAGTTCCTCTAACAACGATGCTGCAGGCAATGGTGCTGCTCAAACTTTT
GGCGGATACTCTGTTGCTGATCTTCAACAACGTTACAACACCGTATATTTTGGT
TTTGATAAATACGACATCACCGGTGAATACGTTCAAATCTTAGATGCGCACGCA
GCATATTTAAATGCAACGCCAGCTGCTAAAGTATTAGTAGAAGGTAATACTGAT
GAACGTGGTACACCAGAATACAACATCGCATTAGGACAACGTCGTGCAGATGCA
GTTAAAGGTTATTTAGCAGGTAAAGGTGTTGATGCTGGTAAATTAGGCACAGTA
TCTTACGGTGAAGAAAAACCTGCAGTATTAGGTCACGATGAAGCTGCATATTCT
AAAAACCGTCGTGCAGTGTTAGCGTACTAA

SEQ. ID NO:25
Amino acid sequence of P6 from non-typeable H. influenzae.

>p6nthipatent.PRO
MNKFVKSLLVAGSVAALAACSSSNNDAAGNGAAQTFGGYSVADLQQRYNTVYFG
FDKYDITGEYVQILDAHAAYLNATPAAKVLVEGNTDERGTPEYNIALGQRRADA
VKGYLAGKGVDAGKLGTVSYGEEKPAVLGHDEAAYSKNRRAVLAY.

SEQ. ID NO:26
Nucleotide sequence of the coding region of pcp from H. influenzae strain HiRD.

PCP hird

ATGAAAAAAACAAATATGGCATTAGCACTGTTAGTTGCTTTTAGTGTAACTGG
TTGTGCAAATACTGATATTTTCAGCGGTGATGTTTATAGCGCATCTCAAGCAA
AGGAAGCGCGTTCAATTACTTATGGTACGATTGTTTCTGTACGCCCTGTTAAA
ATTCAAGCTGATAATCAAGGTGTAGTTGGTACGCTTGGTGGTGGAGCTTTAGG
TGGTATTGCTGGTAGTACAATTGGCGGTGGTCGTGGTCAAGCTATTGCAGCAG
TAGTTGGTGCAATTGGCGGTGCAATAGCTGGAAGTAAAATCGAAGAAAAAATG
AGTCAAGTAAACGGTGCTGAACTTGTAATTAAGAAAGATGATGGTCAAGAGAT
CGTTGTTGTTCAAAAGGCTGACAGCAGTTTTTGTAGCTTGGTCGCCGAGTTCG
TATTTGTTGGTGGCGGCTCAAGCTTAAATGTTTCTGTGCTA

SEQ. ID NO:27
Amino acid sequence of pcp from H. influenzae strain HiRD.

MKKTNMALALLVAFSVTGCANTDIFSGDVYSASQAKEARSITYGTIVSVRPVK
IQADNQGVVGTLGGGALGGIAGSTIGGGRGQAIAAVVGAIGGAIAGSKIEEKM
SQVNGAELVIKKDDGQEIVVVQKADSSFCSLVAEFVFVGGGSSLNVSVL

TolQ 22100-22789 below SEQ ID NO:28 - Moraxella catarrhalis
TolR 22815-23250 below SEQ ID NO:30 - Moraxella catarrhalis
TolB 24097-25359 below SEQ ID NO:34 - Moraxella catarrhalis
TolX 23253-24080 below SEQ ID NO:32 - Moraxella catarrhalis

Also the sequence 21051-25650 is a further nucleotide sequence of the
invention, particularly the 1000bp region upstream of the TolQ gene
initiation codon.

MCA1C0024  Length: 33248  Type: N  Check: 1253 ..

21051 GGGTGATAGC GCACCTCAAC AGGATAGCTA CGACCCTCGA CAATATACAC

21101 AGGTGCAGGT TTGCCATTCG CTGCAAAATA GTCAGAAAAC CTTTGGGTGT

21151 CTAAAGTGGC GGAGGTGATG ATAACTTTTA GATCAGGGCG TTTGGGTAAA

21201 AGACGCTTTA AATAGCCCAT GATAAAATCA ATATTTAAGC TACGCTCATG

21251 TGCTTCATCA ATGATGATGG TATCATAATT TGCCAAAAAC TTATCAGAGC

21301 CCAATTCAGC AAGTAAAATC CCATCTGTCA TCAGCTTGAC AATAGAGTGC

21351 TTGCCACCTT CTTCGGTGAA GCGAATCTTA AAACTCACCG TCTGACCAAG

21401 TGGCTCGCCA AGCTCTTCAG CGATACGCAT CGCTACCGAG CGTGCAGCCA

21451 ATCGGCGTGG CTGTGTGTGG CCAATTTGAC CTGTGATGCC ACGCCCTGCC

21501 ATCATAGCAA GCTTAGGCAG TTGCGTGGTT TTGCCAGAAC CCGTCTCACC

21551 TGCGATAATC ACCACTTGAT GATCACGGAT CGCTTGAATT AGCGTATCGG

21601 CTTCAGCAGT CACGGGCAAA TCATGATTAA GTTTTTCTGA TAGATTTTTT

21651 GGTATGCTAT CCATACGATT GGCGACCTGT TCGGCAGATC GCTCATAGAT

21701 AGCATCATAG CGTATTTTGC ACTTAGTTTT TAGATCGCCT GTGGTAGAAT

21751 TCATTTTCTG TTTTAGTTTA TTTAAATAAT GTCTGTCTTT GGCAAGGACT

21801 GGTAAATTAT CGGTAGAATG CATATTTTTA AATGATAGTT ATCTTATAAA

21851 GGGTATGAAA AAGCATCAAT TTAAGTACAT TGATACATCA GATTTTATTT

21901 TATTCATGGG TCTATATGAG GGCTTGGACG CATGAATAAA CCATGTATTG

21951 TAAATAAAAT CATCAAAACC TGCAATTTTC TATTTAAATG GCGATTTTAG

22001 GGCGATAGAC AAGCGATGAC TTTTTGCCCA TCTGTCGCAA ATTTATTAAC

22051 TTATGCTATA ATGCCAAGTA TCTTTTTTTG CCTATTGTGA TTGTCAATTA

22101 TGAACGAATC CATTAGCCTA ATCTCGCTGG TCATTGAAGC AAGCGTTGTT

22151 GTTAAATTGG TCATGGCGAT ACTGCTTTTG CTGTCTACAA TCAGTTGGGT

22201 ACTGATTTTT CATCTGGGTA CCAAAATTGG CGGTATTGCC AAGTTTGATA

22251 AGCGATTTGA GCGATGGTTT TGGACTGATG ATATCGATCA TCAGCTGTCT

22301 GTTGTGCAAG CAGAATCAGA GCGTGCAGGG CTTGAGCTGA TTTTTTATAC

22351 AGGTTTTTAT GATCAAAATC ACCAAGACCA AGATTCTTCA CTAAGTGATG

22401 ATAAAAAAGT GCAAATCGTT GAGCGTCGCT TGCGTATGGC ATTAGGCAGT

22451 GAGCAGGTGC ATCTTGAAAA AGGATTATCA ACGCTTGCAA CGATTGGTTC

22501 TGTTTCACCT TATATCGGAC TATTTGGTAC AGTATGGGGC ATTATGAATG

22551 CATTTATTGG CTTGGGTCAA GCCGAATCGG TTGGTCTTGC AACCGTTGCA
22601 CCGAGCATTG CTGAGGCATT GATTGCAACA GCACTTGGTT TATTTGCGGC
22651 CATTCCTGCG ACGATGGCAT ATAATCACTT TGCCACCAAA TCCAATACAC
22701 TGTATGAAAA TCGTAGCCTA TTTTGTGAAG GCTTAATAAG TGCATTGGTG
22751 ACAAATCTGG CAAAAAAGAA CACCGCATCA ACTTTATAGA GCATACTATT
22801 TTATAGAGCA TATTATGGTA ACTTCCAATC GATTCGCTCG TCGCCAAAGA
22851 CCGCTAAATA GTGACATGAA TGTTGTGCCT TACATTGATG TGATGTTGGT
22901 GCTTTTGGTG ATATTTATCG TAACAGCACC AATGCTTGCT ACAGGTATTG
22951 AGGTATCACT GCCAAAAGAG CAGACCAAAC CCATCACACA AGCTGACAAG
23001 CTGCCTGTCA TTGTCAGCAT TCAGGCAGAT GGCAATCTGT ATGTCAGCCA
23051 TAAAAATGCC ATCGATGTGC CAATCACGCC TGACAAGCTA GATACCCTGC
23101 TACGCCAGAT GCACCAAGAC AATACCGATT TACAAGTGAT GGTCAATGCC
23151 GATGCAGATA ATGCCTACAG CCGAATTATG CAGATTATGG CATTGATTCA
23201 AAATGTTGGT ATCACCCAAG TGAGTTTGCT TAGCGAATCT GTTCAATAAT
23251 GCATGATAAT TCATAAGGCA AATCAATCGA TGCGTTTATC CGATAATCAT
23301 CCAACAGTCA ATTTTGATAA ATCTGCGCTA ATTTTACCAA TTTTAGCCAG
23351 TGTTTTATTA CATACCGTCA TCATCATAGC GGTAGCAGCA CCACTGATTA
23401 CACCGCCTAC TAAGCCTAAT ACTACTATTC AGACCGCTTT GGTAGGTCAA
23451 GAGGCTTTTA ATCGTGCCAA GACGGCCTTG AGCAATCATC ATGCCAATCA
23501 AAACAAGCCA ACTGCCACCA ACACTTCAAG TACCATCACT GCCAATGATA
23551 ATGATAATGC ATTTATGCAA GCTCAAAATC AGCATCGTTA TCACCCACAG
23601 GTTTCTACTT CTGCCACCAC GACCCAAGCG TATCATCCAC CACCCAACTC
23651 AGCACCCTTT GAATCAAATT CACCAAATAT ACAAAATCAA CCAACAAACG
23701 CTCACGCCAA GCTGGCTGAA TATTCTAATC ATGTCTCAGA CCTTGAGCAG
23751 TCAAATCATA CCGAGTCTAC GCCAAGCCGA GCACAAATCA ATGCCGCCAT
23801 CACCTCGGTC AAACATCGTA TTGAAGCCAT TTGGCAACGC TATCCTAAGC
23851 AGCCCAATCA AACCATCACC TTTCAGGTTA ATATGAATCA ACAAGGCGAT
23901 GTGACCTCAA TCCAATTCGG TGGTGGCCAT CCTGATTTGC GTGAATCTGT
23951 AGAAGCGGCG GTATATGCTG CCGCACCATT TTATGAACTT GGCGGTATGC
24001 GTGACAGTAT CCGCCTGCAG TTCACCACAG AGCAGCTAAT TATGGATAAT
24051 AACCAAACAA CCAATGAGCC TAATCACTAA TCGCCATGGA GTTTTTATGA
24101 AATCACCCAT TACCAAAGTT TGCCTTGCTC TGACCATAAG CTTTTCTGCC
24151 GCTTTGACGC ACACTTATGC TGATGATGAA TTGATTGTGA TTAGCGAACA

24201 AGTTGCTCCG AGTCAATACC CCGTGGCAGT CATGCCTTTT TCAGAAGCTC

24251 ATCAAATGAG TCATTATCTA AGCCTGGCAG GTCTTGGTAC TACTCACCAA

24301 AACCTGCCAC AGCACACTCA GACGAATAGC GACATTCTGA ATAATCTGAC

24351 CGCATGGCGT AACCGAGGAT TTGAATATAT TATTTTGGCA CAGTCGCATC

24401 AAATTTTGGG AAATAAGCTT GCAATTAACT ATGAAATTAT TGATACTGCC

24451 AATGGTTTGG TAAGCGTCAA GCATACCCAA ATTAGCGATA ACCACCCTGC

24501 TTCTATCCAA GCTGCCTATC GTCAAATCAG CGATACAATC TATCAAATCA

24551 TCACAGGCCA GCCATCAGAT TTGATGGGTA AAATCGCCTA TGTGGAAGAA

24601 AGCGGATCGC CACAAAATAA AATCTCATCT CTTAAATTGA TTGATCCAAG

24651 CGGTCAGCTT ATCCGTACGC TAGATACCGT CAATGGATCA ATTATAACGC

24701 CGACATTTTC CCCCGATGGC TTGAGTATTG CTTATAGTGT ACAAACAAAA

24751 AATAATCTGC CCATCATTTA TATTGTGTCT GTATCAGGTG GCACACCAAA

24801 GCTCGTCACG CCATTTTGGG GTCATAATTT GGCACCAAGT TTTTCACCAG

24851 ATGGTAGCAG TATCTTATTT TCAGGTAGCC ACGAGAATAA TAACCCGAAC

24901 ATTTATCGTC TTAATTTACA TACCAATCAC TTAGATACGC TCACTACATT

24951 CAACGGTGCT GAGAATGCAC CAAATTATTT GGCAGATGCG TCAGGATTTA

25001 TTTATACTGC TGATAAAGGT ACACGCCGCC AAAGCCTATA TCGCTATGAT

25051 TTTGGCACGA CGCATAGCAC CCAAATCGCC TCTTATGCCA CCAATCCACG

25101 CTTAAGCCCA GATGGATCAA AGCTTGTATA TTTATCAGGT GGACAAATCA

25151 TCATCGCCAA TACCAAAGGC CGTATCCAAC AAAGTTTTAG GGTGTTAGGC

25201 ACTGATGTAT CAGCCAGCTT TTCACCATCA GGCACACGGA TTATATATAC

25251 ATCCAACCAA GGCAATAAAA ACCAGCTGAT GATCCGTTCG CTATCAAGTA

25301 ATGCCATACG CACCATCCCA ACATCAGGCA CGGTGCGTGA TCCGATTTGG

25351 TCAAAATAAT GCCAATGAGT ATCCCAACTA AGGCGACAGT CGGCTATACC

25401 CAAAGGCGGT TATTTATGGT CAGTATGACA GTTGGCCTGA TCAGCTTGAG

25451 TGGGTGTCAG CACATTCAAG TGACCAAAAG CCCAATACCG ATCATCATCC

25501 ATAGCCATAC AAAATCGCCA TCTCAGCCTA AACCTACACC AACTGACGCC

25551 GTGCCTACCA AAAACCGCCC AATCTCCCCA CCAACACAAA AGTCCAATAC

25601 GATATTTATT TTGGAAGATT GGTTTTAGGC AGTTTTGGTA GATTCAAAAT

SEQ ID NO: 29 - amino acid sequence of TolQ from M. catarrhalis
!!AA_SEQUENCE 1.0
TRANSLATE of: contig24.txt check: 1253 from: 22100 to: 22786
generated symbols 1 to: 229.

MCA1c0024

tolQ-Mcat.pep  Length: 229  December 22, 1999 09:12  Type: P  Check:
2526  ..

1  MNESISLISL VIEASVVVKL VMAILLLLST ISWVLIFHLG TKIGGIAKFD

51  KRFERWFWTD DIDHQLSVVQ AESERAGLEL IFYTGFYDQN HQDQDSSLSD

101  DKKVQIVERR LRMALGSEQV HLEKGLSTLA TIGSVSPYIG LFGTVWGIMN

151  AFIGLGQAES VGLATVAPSI AEALIATALG LFAAIPATMA YNHFATKSNT

201  LYENRSLFCE GLISALVTNL AKKNTASTL

SEQ ID NO: 31 - amino acid sequence of TolR from M. catarrhalis
!!AA_SEQUENCE 1.0
TRANSLATE of: contig24.txt check: 1253 from: 22815 to: 23246
generated symbols 1 to: 144.

MCA1c0024

tolR-Mcat.pep  Length: 144  December 22, 1999 09:13  Type: P  Check:
507  ..

1  MVTSNRFARR QRPLNSDMNV VPYIDVMLVL LVIFIVTAPM LATGIEVSLP

51  KEQTKPITQA DKLPVIVSIQ ADGNLYVSHK NAIDVPITPD KLDTLLRQMH

101  QDNTDLQVMV NADADNAYSR IMQIMALIQN VGITQVSLLS ESVQ

SEQ ID NO: 33 - amino acid sequence of TolX from M. catarrhalis

MIIHKANQSMRLSDNHPTVNFDKSALILPILASVLLHTVIIIAVAAPLITPPTKPNTTIQTALVGQEAF
NRAKTALSNHH
ANQNKPTATNTSSTITANDNDNAFMQAQNQHRYHPQVSTSATTTQAYHPPPNSAPFESNSPNIQNQPTN
AHAKLAEYSNH
VSDLEQSNHTESTPSRAQINAAITSVKHRIEAIWQRYPKQPNQTITFQVNMNQQGDVTSIQFGGGHPDL
RESVEAAVYAA
APFYELGGMRDSIRLQFTTEQLIMDNNQTTNEPNH

SEQ ID NO: 35 - amino acid sequence of TolB from M. catarrhalis

!!AA_SEQUENCE 1.0
TRANSLATE of: contig24.txt check: 1253 from: 24097 to: 25356
generated symbols 1 to: 420.

MCA1c0024

tolB-Mcat.pep  Length: 420  December 22, 1999 09:08  Type: P  Check:
3135  ..

1  MKSPITKVCL ALTISFSAAL THTYADDELI VISEQVAPSQ YPVAVMPFSE

51  AHQMSHYLSL AGLGTTHQNL PQHTQTNSDI LNNLTAWRNR GFEYIILAQS

101 HQILGNKLAI NYEIIDTANG LVSVKHTQIS DNHPASIQAA YRQISDTIYQ

151 IITGQPSDLM GKIAYVEESG SPQNKISSLK LIDPSGQLIR TLDTVNGSII

201 TPTFSPDGLS IAYSVQTKNN LPIIYIVSVS GGTPKLVTPF WGHNLAPSFS

251 PDGSSILFSG SHENNNPNIY RLNLHTNHLD TLTTFNGAEN APNYLADASG

301 FIYTADKGTR RQSLYRYDFG TTHSTQIASY ATNPRLSPDG SKLVYLSGGQ

351 IIIANTKGRI QQSFRVLGTD VSASFSPSGT RIIYTSNQGN KNQLMIRSLS

401 SNAIRTIPTS GTVRDPIWSK

SEQ. ID NO:36
Nucleotide sequence of the coding region of tolA from M. catarrhalis.

TolA nucleotides 27473-28852 in the sequence below
Also the sequence 26451-28900 is a further nucleotide sequence of the
invention, particularly the 1000bp region upstream of the TolA gene
initiation codon.
!!NA_SEQUENCE 1.0

MCA1C0028 Length: 49617 Type: N Check: 3684 ..

26451 GGCGACTGGC GGATTGTGGA GTATCGCTGT ACTGTGTACT CATTGCACCC

26501 ATGGCATCAA ACATACACGA TTGCGTCCAA TGCTCACTTT CACCGCCGCC

26551 TGCCAGTACG ATATCAGCCT TACCAAGTTG AATCAGCTCC ATGGCATGAC

26601 CGACACAGTG GCTTGAAGTG GCACAGGCAG AAGATAGCGA GTAAGACAAG

26651 CCCTTGATTT TTAGCCCCGT CGCTAAGGCC GCGGATACCG AGCTTGCCAT

26701 GATTTTGGGA ACTGCCATTG CACCTACGCC ACGCAAGCCT TTTTCACGCA

26751 TGGCATCCGC AGCTGCCACC ACATCCGCAG TAGAAGCACC GCCCGATGCT

26801 GCAACCACCG AAACCCTAGG ATTGTCAGTG ATGGTGTCAA TGCTAAGCCC

26851 TGCGTTTTTG ATTGCTGATA AAGCACTGAT ATATGCATAA AGGCTGGCGT

26901 TGCTCATAAA GCGCTTTAAT TTACGATCAA TGCCTGTGGT GTCCAAGTCA

26951 TCATGATCTA TACTACCTGC CACGCATGAT TTAAATCCCA AATCGGCATA

27001 TTCTTGCTTA AAGCGAATGC CTGAACGCCC ATTTTCTAAG GCCTCCTTGA

27051 CGGTATCTAA ATCATTACCC AAGCAAGAAA CAATGCCTGC ACCTGTGATG

27101 ACAACTCGTT TCATAATTTC ATCCTAAAAA GTTTACAGTT GTAATCTTGC

27151 TATTGTAACA AATTATTCCA ACACTTAGGG AAATTTTCCC AAAATTTTCA

27201 TAAAAATAGG TGAAAATGAC TAAAGATAGA CAAGGGTTTA CCAAATATTT

27251 AGTTATTCAT CAATTGGCGA CGGTATTTAT GAACATTTAA TAACATTTAT

27301 GTTGTATATT ATCACTAGGC GTAGTTTAGT TTTTGTGATA ATCTTTAGAA

27351 GATAATTTTT ATGACAATTT CATACAATTA ATGAGGTTGG ACATACGATA

27401 GATAAAAGTA AATTGACTTT TTGTATTTTA TGTCAAAACC TGAATCTTAA

27451 TACCAAAATC ATGGAGTAAC TGATGACAAA ATCAACTCAA AAAACCACCA

27501 AACAAACACA ACACAGCCAT GATGATCAAG TCAAAGAGCT GGCTCAAGAA

27551 GTCGCTGAAT ATGATGATGT TGAAATTGTT GCTGAAGTAG ATATCGACAA

27601 TCAAGCTGTC TCTGATGTTT TGATTATTCG TGATACGGAT ACCAAAGCTG

27651 ACCAAGCAGA TCACACTGAT GACGCATCTA AAGCAGATGA TGAGACTGTG

27701 GTAGATGGCG TTAAACAAAA AGCTCAAGAG GCTAAAGAAG ATTTTGAAAA

27751 TAAAGCACAA GATCTTCAAG ATAAAGCTAC TGAGAAGCTT GAAGTCGCCA

27801 AAGAAGCTAC CCAAGACAAG GTAGAGAAAA CTCAAAGTTT AGTTGAGGAT

27851 ATCAAGGATA AAGCCCAATC TTTGCAAGAA GATGCTGCCG ATACAGTTGA

27901 AGCGTTAAAA CAAGCGGCCA GTGATAAGGT TGAGACTACC AAAGCTGAAG

27951 CTCAATCACT AAAAGATGAT GCTACTCAAA CATTTGAATC AGCCAAACAA

28001 GCGGTTGAAG GCAAAGTAGA AGCCATCAAA GAGCAAGTCT TAGATCAGGT

28051 TGACTCCCTA AAAGACGATA CCGATCAAGA TAATACTGAT CAAGATCAAG

28101 AAAAACAGAC CCTAAAAGAT AAGGCGGTGC AAGCTGCCAC CGCTGCTAAA

28151 CGCAAAGTTG AAGATGTGGT AGATGATGTC AAACACACCA CCGAATCTTT

28201 CAAAAATACC GCAAGCGAAA AAATAGATGA GATTAAGCAA GCTGCTGTTG

28251 ACAAAACAGA AGAGGTCAAA TCTCAGCTTA GCCAAAAAGC TGATGCCCTA

28301 AAATCTTCTG GCGAAGAACT CAAGCAAACA GCTCAAACGG CTGCTAATGA

28351 TGCCATTACA GAGGCTCAAG CTGCCGTAGT AAGTGGTTCG GTTGCTGCCG

28401 CTGATTCGGC ACAATCAACC GCTCAAAGTG CAAAAGATAA GCTCAATCAG

28451 CTCTTTGAAC AAGGTAAGTC CGCTTTGGAT GAAAAAGTTC AAGAATTGGG

28501 CGAGTAATAT GGTGCAACTG AGAAAATTAA TGCAGTCAGC GAATATGTAG

28551 ATCTGGCTAC CCAAGTCATT AAAGAAGAAG CACAAGCACT ACAAACCAAT

28601 GCCCAAGAAT CTCTACAAGC TGCCAAAGCG GCTGGCGAAG AGTATGACGC

28651 TACCCACGAA GATAAGGGTT TGACCACTAA ACTTGGTACA GTGGGTGCCT

28701 ATTTGTCTGG CATGTATGGC ATTAGCCAAA ATAAAAATAA CCATTACCAA

28751 GGCGTTGACT TGCATCGTGA AAGTTTTGAT AAAGATGCAT TTCATGCCCA

28801 AAGCAGTTTT TTTGCAGGGA CAAATATTTG GTGCCAAAGC AGTTGCAGCT

28851 AAGAATGTGG CAGCTAAAGT TGTTCCTCAA TCTAAATTTG AAGCCATCGG

SEQ ID NO: 37 - amino acid sequence of TolA from Moraxella
catarrhalis

!!AA_SEQUENCE 1.0
TRANSLATE of: contig28.txt check: 3684 from: 27473 to: 28849
generated symbols 1 to: 459.

MCA1c0028

tolA-Mcat.pep Length: 459 December 22, 1999 09:05 Type: P Check: 8307 ..

1 MTKSTQKTTK QTQHSHDDQV KELAQEVAEY DDVEIVAEVD IDNQAVSDVL

51 IIRDTDTKAD QADHTDDASK ADDETVVDGV KQKAQEAKED FENKAQDLQD

101 KATEKLEVAK EATQDKVEKT QSLVEDIKDK AQSLQEDAAD TVEALKQAAS

151 DKVETTKAEA QSLKDDATQT FESAKQAVEG KVEAIKEQVL DQVDSLKDDT

201 DQDNTDQDQE KQTLKDKAVQ AATAAKRKVE DVVDDVKHTT ESFKNTASEK

251 IDEIKQAAVD KTEEVKSQLS QKADALKSSG EELKQTAQTA ANDAITEAQA

301 AVVSGSVAAA DSAQSTAQSA KDKLNQLFEQ GKSALDEKVQ ELGE*YGATE

351 KINAVSEYVD LATQVIKEEA QALQTNAQES LQAAKAAGEE YDATHEDKGL

401 TTKLGTVGAY LSGMYGISQN KNNHYQGVDL HRESFDKDAF HAQSSFFAGT

451 NIWCQSSCS

SEQ. ID NO:38
Nucleotide sequence of the coding region of OmpCD from Moraxella catarrhalis.

Omp CD Mcat DNA

ACCESSION L10755
atgaaatttaataaaatcgctcttgcggtcatcgcagccgttgcagctccagttgcagctccagttgct
gctcaagctgg
tgtgacagtcagcccactactacttggctatcattacactgacgaagcccacaatgatcaacgcaaaat
cttacgcactg
gcaagaagctagagctagatgctactaatgcacctgcaccagctaatggcggtgtcgcactggacagtg
agctatggact
ggtgctgcgattggtatcgaacttacgccatcaactcagttccaagttgaatatggtatctctaaccgt
gatgcaaaatc
ttcagacaaatctgcacatcgctttgatgctgagcaagaaaccatcagcggtaactttttgattggtac
tgagcagttca
gcggctacaatccaacaaataaattcaagccctatgtcttggttggtgcaggtcaatctaaaattaaag
taaatgcaatt
gatggttatacagcagaagtagccaatgggcaaaacattgcaaaagatcaagctgtaaaagcaggtcaa
gaagttgctga
gtctaaagacaccatcggtaacctaggtcttggtgctcgctacttagtcaatgatgcccttgcacttcg
tggtgaagccc
gtgctatccataattttgataacaaatggtgggaaggcttggcgttggctggtttagaggtaactttgg
gtggtcgtttg
gcacctgcagtaccagtagcaccagtggcagaacctgttgctgaaccagttgttgctccagcacctgtg
atccttcctaa

accagaacctgagcctgtcattgaggaagcaccagctgtaattgaagatattgttgttgattcagacgg
agatggtgtgc
ctgatcatctggatgcctgcccaggaactccagtaaacactgttgttgatccacgcggttgcccagtac
aggttaatttg
gtagaagagcttcgccaagagttgcgtgtattctttgattatgataaatcaatcatcaaaccacaatac
cgtgaagaagt
tgctaaggttgctgcgcaaatgcgtgaattcccaaatgcaactgcaaccattgaaggtcacgcatcacg
cgattcagcac
gctcaagtgcacgctacaaccagcgtctatctgaagctcgtgctaatgctgttaaatcaatgctatcta
acgaatttggt
atcgctccaaaccgcctaaatgcagttggttatggctttgatcgtcctatcgctccaaatactactgct
gaaggtaaagc
gatgaaccgtcgtgtagaagcagtaatcactggtagcaaaacaacgactgttgatcaaaccaaagatat
gattgttcaat
aa

SEQ. ID NO:39
Amino acid sequence of OmpCD from Moraxella catarrhalis

Peptide
MKFNKIALAVIAAVAAPVAAPVAAQAGVTVSPLLLGYHYTDEAHNDQRKILRTGKKLELDATNAPAPAN
GGVALDSELWT
GAAIGIELTPSTQFQVEYGISNRDAKSSDKSAHRFDAEQETISGNFLIGTEQFSGYNPTNKFKPYVLVG
AGQSKIKVNAI
DGYTAEVANGQNIAKDQAVKAGQEVAESKDTIGNLGLGARYLVNDALALRGEARAIHNFDNKWWEGLAL
AGLEVTLGGRL
APAVPVAPVAEPVAEPVVAPAPVILPKPEPEPVIEEAPAVIEDIVVDSDGDGVPDHLDACPGTPVNTVV
DPRGCPVQVNL
VEELRQELRVFFDYDKSIIKPQYREEVAKVAAQMREFPNATATIEGHASRDSARSSARYNQRLSEARAN
AVKSMLSNEFG
IAPNRLNAVGYGFDRPIAPNTTAEGKAMNRRVEAVITGSKTTTVDQTKDMIVQ

SEQ. ID NO:40
Nucleotide sequence of the coding region of xOmpA from Moraxella catarrhalis

xompa

!!NA_SEQUENCE 1.0
MCA1C0035

mca1c0035.seq  Length: 2461  December 2, 1999 11:22  Type: N  Check:
9214  ..

1  ATGTGTTTGC ATTGATTGAT AAATACACGC TTAGTCTAGC AGATTTTTGG

51  TAAAATGCTT AGCCTTTGTA CGATTTTATG GCTAATTTTA ATAACAAGTG

101  AATAAAAACT ACCAACTTTT TGGTAAATTT GATTTTAAGT ATAAGTGGTT

151  CATGTAATTT ATATGCCAAA AAGTATGTGC ATAAAATCAA TCAAATGGTT

201  TATCTGTCAA TTTGATGAGT GGGTATTGAG GGTTTTTGCT TCATGATTAA

251  AATCATTGAG AATTAATTAC TATCATAATT ACTATAATAT TACAGATATG

301  TAAATAAAAA ACCATTCATC ATTTACTTTT GTAATTGCTT AATTTTTTTT

351  GAGCGAATAA AAGGCGGTTT TGTTTATCAA TTGTTGCCAG CGCTTTTAAG

401  TTGCCATAAA ATCAGTCACA ATAGAGTTAT AAAACAAGTG GCTTCAAGCA

451  ACTTGTTGTT TTTCTTAAGG ACGGCATCGG CATTTTGCTG ATGGATAATG

501 AAATTTAAAT TTAAAATGAC CTATGGAGTG ACTTATGAGC TTAATTAATA
551 AATTAAATGA ACGCATTACG CCGCATGTCT TAACTTCGAT TAAAAATCAA
601 GATGGCGATA ATGCTGATAA ATCTAATTTG TTAACCGCAT TTTATACCAT
651 TTTTGCAGGA CGCTTGAGTA ATGAAGATGT GTATCAGCGT GCCAATGCTT
701 TGCCTGATAA TGAGCTTGAG CATGGGCATC ATCTGCTCAA TGTTGCTTTT
751 AGTGATGTTT CAACTGGTGA AGATCAGATT GCTTCTTTGA GTAATCAATT
801 AGCCGATGAA TATCATGTTT CGCCAGTAAC GGCACGCACC GCAATCGCAA
851 CGGCAGCACC TTTGGCTTTG GCACGCATTA ACATTAAAGA GCAAGCAGGT
901 GTATTGTCTG TACCGTCTTT TATTCGTACT CAATTGGCTA AAGAAGAAAA
951 CCGTTTGCCA ACTTGGGCGC ATACTTTATT GCCAGCAGGG CTATTTGCAA
1001 CCGCTGCCAC AACCACCGCC GAGCCTGTAA CGACAGCCTC TGCTGTTGTG
1051 AAAGAGCCTG TCAAACCAAG TGTTGTGACA GAACCAGTTC ATCCAGCTGC
1101 GGCTACCACC CCAGTCAAAA CACCAACTGC CCGGCATTAC GAAAACAAAG
1151 AAAAAAGTCC TTTTCTAAAA ACGATTCTAC CGATTATTGG ATTGATTATT
1201 TTTGCAGGCT TGGCATGGCT TTTGTTAAGA GCATGTCAAG ACAAACCAAC
1251 ACCTGTTGCG GCACCTGTTG CGACAGATAC AGCACCTGTG GTAGCGGATA
1301 ATGCTGTACA GGCAGACCCA ACACAAACAG GTGTTGCCCA AGCACCTGCA
1351 ACGCTTAGCT TGTCTGTTGA TGAAACGGGT CAAGCGTTGT ACTCGCACCG
1401 TGCTCAGGTT GGTAGTGAAG AGCTTGCAGG TCATATCCGT GCAGCTATTG
1451 CTCAAGTCTT TGGCGTACAA GATTTAACCA TTCAAAATAC CAATGTACAT
1501 ACCGCTACGA TGCCAGCGGC AGAATACTTA CCAGCAATTT TGGGTTTGAT
1551 GAAAGGTGTA CCAAATTCAA GCGTTGTGAT TCATGATCAT ACGGTACGCT
1601 TTAATGCAAC CACGCCAGAA GATGTAGCAA AACTGGTAGA GGGTGCTAAA
1651 AATATTCTAC CCGCTGATTT TACTGTAGAA GCAGAACCTG AACTTGATAT
1701 TAATACTGCG GTTGCCGATA GTATTGAAAC AGCGCGTGTT GCTATTGTTG
1751 CTTTGGGTGA TACGGTTGAA GAAAATGAGA TGGATATTTT AATCAATGCA
1801 TTAAATACCC AAATCATTAA CTTTGCTTTA GACTCAACCG AAATTCCCCA
1851 AGAAAATAAA GAAATCTTGG ATTTGGCTGC CGAAAAATTA AAGGCAGTGC
1901 CTGAAACAAC TTTGCGTATC ATTGGTCATA CAGACACTCA AGGCACGCAT
1951 GAGTATAATC AAGATTTATC AGAATCTCGT GCTGCTGCTG TTAAAGAGTA
2001 TTTGGTATCA AAAGGTGTTG CTGCTGAACG TTTGAACACT CAAGGTGCAA
2051 GTTTTGATTA TCCAGTTGCA TCAAATGCTA CCGAACAAGG TCGCTTCCAA
2101 AACCGTCGTA TTGAGTTTGT ACTTTTCCAA GAAGGTGAAG CAATTACTCA

2151 AGTCGGTCAT GCTGAAGATG CACCAACACC TGTTGCACAA AACTGATCAT
2201 TTTGTTATTG GTTATGAGTT TTAGATTGGG CCAAATGAAT GATAATATAC
2251 CAATCTTACA AGTACTTTTA ATAACCAAAA CCAACCGTAA TCAACCCAAG
2301 AACCAAATTA CCCATCGGTC ATTTGGTTCT TGGGTAGTTT TTATTGGCTC
2351 TCAATATATG ATGTAGACCA ATTTGACCCA AAATAGATCA GAGTTTGGGT
2401 CTTGGATTTG CGACCATATC GTATAACTGA CATATCTTGA ACACAAAAAA
2451 GCATAAAATG A

SEQ. ID NO:41
Amino acid sequence of xOmpA from Moraxella catarrhalis, and also shown in Fig. 2.

xompa

!!AA_SEQUENCE 1.0
TRANSLATE of: omp854.seq check: 9214 from: 1 to: 2461
generated symbols 1 to: 820.

MCA1C0035

omp854.pep Length: 553 December 2, 1999 11:35 Type: P Check: 5451
..

1 MSLINKLNER ITPHVLTSIK NQDGDNADKS NLLTAFYTIF AGRLSNEDVY
51 QRANALPDNE LEHGHHLLNV AFSDVSTGED QIASLSNQLA DEYHVSPVTA
101 RTAIATAAPL ALARINIKEQ AGVLSVPSFI RTQLAKEENR LPTWAHTLLP
151 AGLFATAATT TAEPVTTASA VVKEPVKPSV VTEPVHPAAA TTPVKTPTAR
201 HYENKEKSPF LKTILPIIGL IIFAGLAWLL LRACQDKPTP VAAPVATDTA
251 PVVADNAVQA DPTQTGVAQA PATLSLSVDE TGQALYSHRA QVGSEELAGH
301 IRAAIAQVFG VQDLTIQNTN VHTATMPAAE YLPAILGLMK GVPNSSVVIH
351 DHTVRFNATT PEDVAKLVEG AKNILPADFT VEAEPELDIN TAVADSIETA
401 RVAIVALGDT VEENEMDILI NALNTQIINF ALDSTEIPQE NKEILDLAAE
451 KLKAVPETTL RIIGHTDTQG THEYNQDLSE SRAAAVKEYL VSKGVAAERL
501 NTQGASFDYP VASNATEQGR FQNRRIEFVL FQEGEAITQV GHAEDAPTPV
551 AQN

SEQ. ID NO:42
Nucleotide sequence of the coding region of P6-like (or PAL-1) from Moraxella catarrhalis

P6-like) Pal mcat DNA
ATGATGTTACATATTCAAATTGCCGCCGCTGCCGCCGCTTTATCGGTACTAACTTTTATG
ACAGGCTGTGCCAATAAATCAACAAGTCAAGTTATGGTTGCTCCTAATGCACCCACAGGT
TACACTGGGGTTATCTATACTGGTGTTGCACCTTTGGTAGATAATGATGAGACCGTTAAG

GCTCTGGCAAGCAAGCTACCCAGTTTGGTTTATTTTGACTTTGATTCTGATGAGATTAAA
CCGCAAGCTGCTGCCATCTTAGACGAACAAGCACAATTTTTAACCACCAATCAAACAGCT
CGTGTTTTGGTTGCAGGTCATACCGATGAGCGTGGTAGTCGTGAGTATAATATGTCACTG
GGGGAACGCCGTGCGGTGGCGGTACGCAACTATTTGCTTGGTAAAGGCATTAATCAAGCC
AGCGTTGAGATTATCAGTTTTGGTGAAGAACGCCCTATCGCATTTGGCACAAATGAAGAA
GCATGGTCACAAAATCGTCGTGCTGAACTGTCTTATTAA

SEQ. ID NO:43
Amino acid sequence of P6-like (or PAL-1) from Moraxella catarrhalis
(P6-like )Pal Mcat peptide
MMLHIQIAAAAAALSVLTFMTGCANKSTSQVMVAPNAPTGYTGVIYTGVAPLVDNDETVK
ALASKLPSLVYFDFDSDEIKPQAAAILDEQAQFLTTNQTARVLVAGHTDERGSREYNMSL
GERRAVAVRNYLLGKGINQASVEIISFGEERPIAFGTNEEAWSQNRRAELSY

SEQ. ID NO:44
Nucleotide sequence of the coding region of PAL-2 from Moraxella catarrhalis

!!NA_SEQUENCE 1.0
Definition : MCat Lipo4 2nd Pal-like lipoprotein  BASB113 SBBMCA012
Accession : BASB113

Lipo4_MCat.seq  Length: 675  April 28, 1999 09:31  Type: N  Check:

LIPO04_MCAT  Length: 675  February 7, 2001 17:42  Type: N  Check:
4424  ..

1  ATGAAAATTA AAGCATTGGG TGTTGTGCTG TTGGCATCAA GTATGGCTTT

51  GGCAGGTTGT GCAAATACAG GCACAACTGG CAATGGCACA GGATTTGGTG

101  GTGCTAATGT CAATAAGGCG GTGATTGGGG CTGTGGCAGG TGCACTTGGC

151  GGTACTGCCA TTTCAAAAGC AACTGGTGGC GAAAAAACAG GTCGTGATGC

201  CATTTTGGGG GCGGCAGTTG GTGCAGCAGC AGGGGCGTAT ATGGAGCGTC

251  AAGCAAAGCA GATTGAGCAA CAAATGCAAG GAACGGGCGT GACTGTAACC

301  CACGATACCG ACACGGGTAA TATTAATCTA ACTATGCCAG GTAATATTAC

351  TTTTGCTCAT GATGACGATA CTTTAAACAG TGCATTTTTG GGTCGTTTAA

401  ACCAGCTGGC TAATACGATG AATCAGTATC ATGAAACAAC GATTGTCATT

451  GTAGGACATA CAGACTCAAC GGGTCAAGCG GCTTATAATC AAGAGCTGTC

501  TGAGCGTCGA GCGGATTCAG TGCGTTATTA CTTGATTAAT CAAGGCGTTG

551  ATCCATATCG TATTCAGACA GTGGGGTATG GTATGCGACA ACCGATTGCA

601  TCGAATGCAA CCGAAGCAGG TCGTGCTCAA AATCGCCGTG TTGAGCTGAT

651  GATTTTAGCA CCGCAGGGTA TGTAA

SEQ. ID NO:45
Amino acid sequence of PAL-2 from Moraxella catarrhalis – see Fig. 2.

Pal2

!!AA_SEQUENCE 1.0

Definition : MCat Lipo4 2nd Pal-like lipoprotein BASB113 SBBMCA012
Accession : BASB113

Lipo4_MCat.pep  Length: 224  April 28, 1999 09:21  Type: P  Check:

LIPO04_MCAT  Length: 224  December 20, 1999 12:28  Type: P  Check:
4279  ..

1  MKIKALGVVL LASSMALAGC ANTGTTGNGT GFGGANVNKA VIGAVAGALG

51  GTAISKATGG EKTGRDAILG AAVGAAAGAY MERQAKQIEQ QMQGTGVTVT

101  HDTDTGNINL TMPGNITFAH DDDTLNSAFL GRLNQLANTM NQYHETTIVI

151  VGHTDSTGQA AYNQELSERR ADSVRYYLIN QGVDPYRIQT VGYGMRQPIA

201  SNATEAGRAQ NRRVELMILA PQGM

## Claims

1. A hyperblebbing Gram-negative bacterium which has been genetically modified by one or more processes selected from a group consisting of: down-regulating expression of one or more *Tol* genes; and attenuating the peptidoglycan-binding activity by mutation of one or more gene(s) encoding a protein comprising a peptidoglycan-associated site.

2. The hyperblebbing Gram-negative bacterium of claim 1 which is selected from the group consisting of *Haemophilus influenzae, Neisseria meningitidis, Neisseria lactamica, Neisseria gonorrhoeae, Helicobacter pylori, Salmonella typhi, Salmonella typhimurium, Vibrio cholerae, Shigella spp., Bordetella pertussis, Pseudomonas aeruginosa* and *Moraxella catarrhalis.*

3. The hyperblebbing Gram-negative bacterium of claim 2 which is a *Haemophilus influenzae* strain which has been genetically modified by down-regulating expression of one or more genes selected from a group consisting of: *tolQ, tolR, tolA* and *tolB.*

4. The hyperblebbing Gram-negative bacterium of claim 2 or 3 which is a *Haemophilus influenzae* strain which has been genetically modified by mutation of one or more genes selected from a group consisting of: *ompP5, ompP6* and *pcp* to attenuate the peptidoglycan-binding activity of the encoded protein(s).

5. The hyperblebbing Gram-negative bacterium of claim 2 which is a *Moraxella catarrhalis* strain which has been genetically modified by down-regulating expression of one or more genes selected from a group consisting of: *tolQ, tolR, tolX, tolA* and *tolB.*

6. The hyperblebbing Gram-negative bacterium of claim 2 or 5 which is a *Moraxella catarrhalis* strain which has been genetically modified by mutation of one or more genes selected from a group consisting of: *ompCD, xompA, pal1,* and *pal2* to attenuate the peptidoglycan-binding activity of the encoded protein(s).

7. The hyperblebbing Gram-negative bacterium of claims 2-6 which has been further genetically engineered by one or more processes selected from the following group: (a) a process of down-regulating expression of immunodominant variable or non-protective antigens, (b) a process of upregulating expression of protective OMP antigens, (c) a process of down-regulating a gene involved in rendering the lipid A portion of LPS toxic, (d) a process of upregulating a gene involved in rendering the lipid A portion of LPS less toxic, and (e) a process of down-regulating synthesis of an antigen which shares a structural similarity with a human structure and may be capable of inducing an auto-immune response in humans.

8. A preparation of membrane vesicles obtained from the bacterium as defined in any one of claims 1-7.

9. The preparation of membrane vesicles of claim 8 which is capable of being filtered through a 0.22 µm membrane.

10. A sterile, homogeneous preparation of membrane vesicles obtainable by passing the membrane vesicles from the bacterium as defined in any one of claims 1-7 through a 0.22 µm membrane.

11. A vaccine which comprises a bacterium as defined in any one of claims 1-7 or a preparation as defined in any one of claims 8-10 together with a pharmaceutically acceptable diluent or carrier.

12. A vaccine according to claim 11 for use in a method of treatment of the human or animal body.

13. A method of protecting an individual against a bacterial infection which comprises administering to the individual an effective amount of a bacterium as defined in any one of claims 1-7 or a preparation as defined in any one of claims 8-10.

14. A process for preparing a vaccine composition comprising a preparation of membrane vesicles as defined in claims 8-9 which process comprises: (a) inoculating a culture vessel containing a nutrient medium suitable for growth of the bacterium of any one of claims 1-7; (b) culturing said bacterium; (c) recovering membrane vesicles from the medium; and (d) mixing said membrane vesicles with a pharmaceutically acceptable diluent or carrier.

15. The process of claim 14 which further comprises a step after either step (c) or step (d), which step comprises sterile-filtering the preparation of membrane vesicles.

16. A method for producing a hyperblebbing bacterium according to claim 1 which method comprises genetically modifying a Gram-negative bacterial strain by one or more of the following processes: (a) engineering the strain to down-regulate expression of one or more *Tol* genes; and (b) attenuating the peptidoglycan-binding activity by mutating one or more gene(s) encoding a protein comprising a peptidoglycan-associated site.
